(19) Europäisches Patentamt
European Patent Office
Office européen des brevets

(11) Veröffentlichungsnummer: **0 323 838 B1**

# (12) EUROPÄISCHE PATENTSCHRIFT

(45) Veröffentlichungstag der Patentschrift: **03.05.95**

(51) Int. Cl.$^6$: **C12N 15/63**, C12N 15/81

(21) Anmeldenummer: **89100105.9**

(22) Anmeldetag: **04.01.89**

## (54) Expressionsvektor und Verfahren zur regulierbaren Herstellung von Proteinen in Eukaryonten.

(30) Priorität: **05.01.88 DE 3800133**
**17.02.88 DE 3804891**

(43) Veröffentlichungstag der Anmeldung:
**12.07.89 Patentblatt 89/28**

(45) Bekanntmachung des Hinweises auf die Patenterteilung:
**03.05.95 Patentblatt 95/18**

(84) Benannte Vertragsstaaten:
**AT BE CH DE ES FR GB GR IT LI LU NL SE**

(56) Entgegenhaltungen:
**EP-A- 0 148 668**

**MOLECULAR AND CELLULAR BIOLOGY, Band 7, Nr. 7, Juli 1987, Seiten 2477-2483, Washington, US; S.H. HONG et al.:**

**CURRENT GENETICS, Band 9, 1985, Seiten 539-545, Berlin, DE; R. RODICIO et al.:**

**MOLECULAR AND GENERAL GENETICS, Band 134, 1974, Seiten 261-272, Berlin, DE; F.K. ZIMMERMANN et al.:**

(73) Patentinhaber: **BOEHRINGER MANNHEIM GMBH**

**D-68298 Mannheim (DE)**

(72) Erfinder: **Kopetzki, Erhard, Dr.rer.nat.**
**Henle-Strasse 2**
**D-8122 Penzberg (DE)**
Erfinder: **Schumacher, Günther, Dr.rer.nat.**
**Kapellenstrasse 20**
**D-8139 Bernried (DE)**
Erfinder: **Zimmermann, Friedrich Karl, Prof. Dr.rer.nat.**
**Goethestrasse 12**
**D-6105 Ober-Ramstadt (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**EP 0 323 838 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**MGG MOLECULAR AND GENERAL GENETICS, Band 200, 1985, Seiten 1-8, Berlin, DE; J.D. COHEN et al.:**

**GENE, Band 41, 1986, Seiten 75-84, Amsterdam, NL; S.H. HONG et al.:**

## Beschreibung

Die Erfindung betrifft eukaryontische Expressionsvektoren, enthaltend einen regulierbaren Promotor insertiert in einen Vektor sowie ein Verfahren zur regulierbaren Herstellung von homologen und heterologen Proteinen oder proteinhaltigen Produkten in eukaryontischen Wirtszellen unter Verwendung eines solchen Vektors.

Die Hefe ist einer der ältesten Kulturorganismen des Menschen. Sie wurde und wird noch immer hauptsächlich zur alkoholischen Gärung (Wein, Bier usw.) und als "Backhilfe" bei der Zubereitung von Teigwaren verwendet. Daneben hat die Hefe industrielle Bedeutung als kostengünstige Rohstoffquelle zur Isolierung von niedermolekularen Substanzen wie z.B. NAD, ATP und Glutathion, und hochmolekularen Substanzen wie z.B. DNA, RNA und vor allem Enzymen wie z.B. Alkoholdehydrogenase, Aldehyddehydrogenase, Acetyl-CoA-Synthetase, $\alpha$-Glucosidase, Glycerinaldehyd-3-phosphat-Dehydrogenase, Glucose-6-phosphat-Dehydrogenase und Hexokinase erlangt.

Sie ist leicht zu kultivieren und aufgrund von langjährigen Erfahrungen im industriellen Maßstab einfach zu fermentieren. Die Hefe, ein zu den niederen Eukaryonten zählender Einzeller, besitzt die typischen Merkmale eines Eukaryonten, ist aber trotzdem genetischen Untersuchungen und genetischen Manipulationen leicht zugänglich, wodurch sie besonders als Wirtsorganismus im Hinblick auf rekombinante DNA-Technologie geeignet ist, d.h. der homologen und heterologen Expression von biologisch aktiven Polypeptiden und Proteinen.

In E. coli, dem bisher am häufigsten benutzten Wirtsorganismus in der rekombinanten DNA-Technologie, unterscheiden sich viele heterolog exprimierte Proteine von ihren in einem homologen System (also für eukaryontische Proteine in einem Eukaryonten) exprimierten natürlichen Gegenstücken in mancherlei Hinsicht und sind biologisch nicht aktiv, oder fallen als unlösliche inaktive Proteinaggregate, "refractile bodies" genannt, an. Viele eukaryontische Proteine, die in E. coli inaktiv exprimiert werden, werden dagegen in Hefe löslich und aktiv gebildet (Biotechnology and Genetic Engineering Reviews 3 (1985) 377-416). Dies mag unter anderem dadurch bedingt sein, daß Hefen über die typischen eukaryontischen posttranslationalen Modifizierungssysteme, wie z.B. "Proteinfaltung", Proteinreifung, Glycosylierung und Acetylierung verfügen, zur Sekretion fähig sind und die Ausbildung von Disulfidbrücken in Polypeptiden und Proteinen ermöglichen. Außerdem sind Hefen nicht pathogen und im Gegensatz zu E.coli frei von Toxinen und pyrogenen Zellwandbestandteilen.

Zur Expression von homologen und heterologen Polypeptiden und Proteinen in Hefen wird üblicherweise eine Expressionskassette benutzt, die aus einem Promotor, dem zu exprimierenden Gen, einem Terminator und gegebenenfalls aus einem oder mehreren den Promotor positiv und/oder negativ beeinflussenden Regulatorgenen besteht. Diese Expressionseinheit befindet sich auf einem hybriden Hefe/E.coli-Vektor ("shuttle vector"). Nach dem Verhalten in Hefe unterscheidet man zwischen 4 Arten von ShuttleVektoren, bezeichnet als YRp, YEp, YIp und YCp. YRp-Plasmide enthalten zur autonomen Replikation in Hefe einen chromosomalen Replikationsursprung aus Hefe, kurz ARS autonomous replicating sequence) genannt, während in YEp-Plasmiden diese Funktionen durch Elemente aus zirkulärer extrachromosomaler Hefe-DNA (2 $\mu$m-DNA) übernommen werden. Sie liegen normalerweise in Stückzahlen von 5 bis 40 Kopien pro Zelle vor. YCp-Plasmide besitzen neben der autonom replizierenden Sequenz ein Centromer und verhalten sich demzufolge wie ein Chromosom. Die YIp-Plasmide enthalten zu chromosomalen Heferegionen homologe Sequenzen und können durch homologe Rekombination in diese Bereiche des Hefechromosoms integriert werden. Sie liegen daher jedoch nur in niedriger Kopienzahl in der Hefezelle vor, was sie zur gentechnologischen Herstellung von homologen oder heterologen Proteinen weniger geeignet macht. Die YRp-und YEp-Plasmide werden ohne Selektionsdruck bei der Zellvermehrung verloren (Nature 305 (1983) 391-397), d.h. plasmidlose Zellen können sich während einer nicht-selektiven Fermentation anreichern, denn sie sind bei ihrer Vermehrung im Vorteil und wachsen schneller, wodurch der Anteil an transformierten Zellen im Zuge der Fermentation ständig abnimmt. Zur Selektion auf plasmidhaltige Hefezellen werden deshalb in die Plasmide Auxotrophiemarker (z.B. URA3, LEU2, TRP1) und Resistenzmarker (z.B. DHFR, CAT, Phosphotransferase) eingebaut, die einen Enzymdefekt in einem essentiellen Biosyntheseweg der verwendeten Hefezelle komplementieren bzw. der Zelle eine Enzymaktivität zur Detoxifizierung von Drogen und Chemikalien (Methotrexat, Chloramphenicol, G 418, Hygromycin B) im Wachstumsmedium verleihen. Bei Verwendung entsprechender defekter Hefestämme oder eines Mediums, das eine bestimmte Chemikalie enthält, wird daher die Vermehrung nicht-transformierter bzw. plasmidloser Zellen blockiert und eine Population überwiegend plasmidhaltiger Zellen erhalten.

Zur Konstruktion von Hefeexpressionsvektoren werden vorwiegend die Promotoren und die dazugehörigen Terminatoren der stark exprimierten glykolytischen Gene wie Hexokinase, Glucoseisomerase, Triosephosphatisomerase, Glycerinaldehyd-3-phosphat-Dehydrogenase, 3-Phosphoglyceratkinase, Enolase, Pyruvatkinase, Pyruvat-Decarboxylase und Alkoholdehydrogenase benutzt. Diese sind jedoch meistens konstitutiv (d.h. sie produzieren gleichbleibende Mengen des Genprodukts) bzw. nur schwach reguliert, was die Expression von für die Zelle toxischen Polypeptiden und Proteine stark beeinträchtigen bzw. unmöglich machen kann. Zur regulierten Expression werden deshalb üblicherweise natürliche regulierte Promotoren, wie z.B. der GAL1-, GAL10-, PHO5-, heat-shock-protein-, MFα1- und CUP1-Promotor verwendet. Zur Kopplung von starker Expression und strikter Regulation (möglichst großes Induktions- bzw. Derepressions- zu Repressionsverhältnis) wurden synthetische Hybridpromotoren entwickelt, die sich aus einem möglichst starken, konstitutiven Promotor, wie z.B. GAP-DH und CYC1-Promotor und einem Regulatorelement (UAS, "upstream activation site") aus dem GAL1,10- bzw. ADHII-Promotor zusammensetzen. Häufig ist die Expressionshöhe und das Induktionsverhältnis dieser Promotoren noch nicht ideal, vor allem, wenn sie sich auf Plasmiden, die in der Zelle in hoher Kopienzahl vorkommen, befinden. Viele dieser Promotoren zeigen Nachteile hinsichtlich einer wirtschaftlichen Induktion im technischen Maßstab. So wird z.B. der PHO5-Promotor durch Phosphatdepletion dereprimiert. Promotoren unter dem Einfluß der "GAL1,10-upstream activation site" benötigen zur maximalen Induktion den teuren Induktor Galactose. Der regulierte MFα1-Promotor wird nur reprimiert in $sir^{ts}$-Mutanten bei 37°C und wird induziert durch eine Temperatursenkung auf 24°C. Andere Promotoren benötigen giftige Schwermetalle wie z.B. Cu, zur Induktion.

Aus praktischen Gesichtspunkten sind daher solche Promotoren besonders gut geeignet, die zum Ende einer Fermentation nach Verbrauch einer billigen C-Quelle entweder dereprimiert werden oder/und durch einen billigen Induktor in der stationären Wachstumsphase bei optimaler Biomasse noch induziert werden können. Dies hat zudem noch den Vorteil, daß viele zelluläre Gene in der stationären Wachstumsphase abgeschaltet werden, wodurch sich die Reinigung eines exprimierten Produktes vereinfachen kann (höhere spezifische Aktivität). Die erhöhte Expression von Proteasen in der stationären Wachstumsphase kann beispielsweise durch die Verwendung von Protease-defizienten Hefestämmen umgangen werden.

Gut regulierte, durch Glucose reprimierbare und durch Maltose induzierbare Hefepromotoren findet man unter den Maltasen (α-Glucosidasen) von S. carlsbergensis (MAL1,2,3,4 und 6) und den nicht näher genetisch charakterisierten Maltasen von S. cerevisiae. Der Vorteil dieser Promotoren ist, daß sie durch billige C-Quellen (Glucose, Maltose) auch im großtechnischen Maßstab kostengünstig zu regulieren sind. Bisher ist aber nur die MAL6S-α-Glucosidase kloniert und deren Promotorsequenz bekannt (Gene 41 (1986) 75-84).

Es zeigte sich jedoch, daß Transformanten, die ein kloniertes α-Glucosidasegen aus S. cerevisiae bestehend aus Promotor, "upstream activation site", Strukturgen und Terminator, selbst nach Induktion mit Maltose und erhöhter Gendosis (d.h. das zu exprimierende Gen liegt auf einem Plasmid vor, das in der Zelle in hoher Kopienzahl vorkommt) nur eine geringe Expression zeigen.

Der vorliegenden Erfindung lag daher die Aufgabe zugrunde, einen Expressionsvektor für die Expression von Proteinen in Eukaryonten zur Verfügung zu stellen, der einen Promotor enthält, dessen Wirkung auf einfache und kostengünstige Weise reguliert werden kann und der eine möglichst effektive Genexpression sowohl in der logarithmischen als auch stationären Wachstumsphase ermöglicht.

Gelöst wird diese Aufgabe durch einen eukaryontischen Expressionsvektor, enthaltend einen regulierbaren Promotor insertiert in eine eukaryontische DNA, welcher dadurch gekennzeichnet ist, daß er als Promotor die DNA-Sequenz der α-Glucosidase pI-Promotor-Region in verkürzter Form enthält, wobei mindestens die gesamte Region upstream des Nukleotids -867 und höchstens die gesamte Region upstream des Nukleotids -386 deletiert ist.

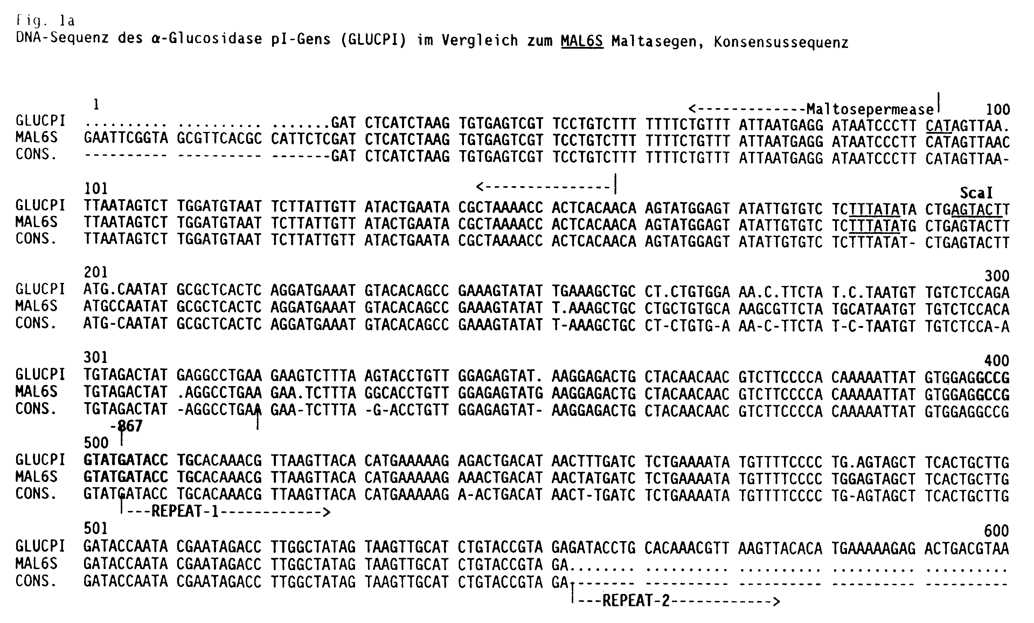

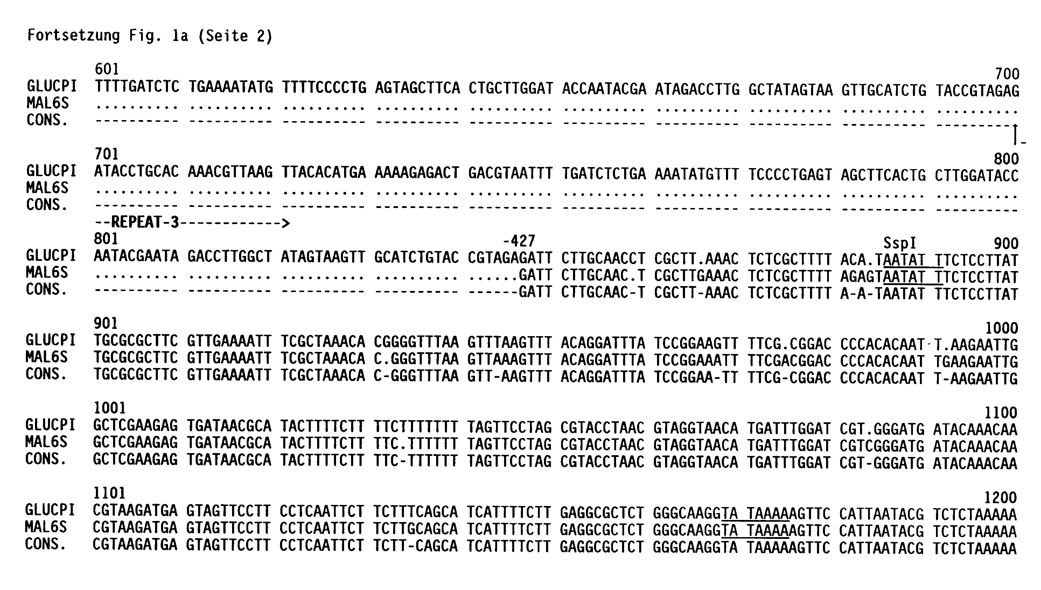

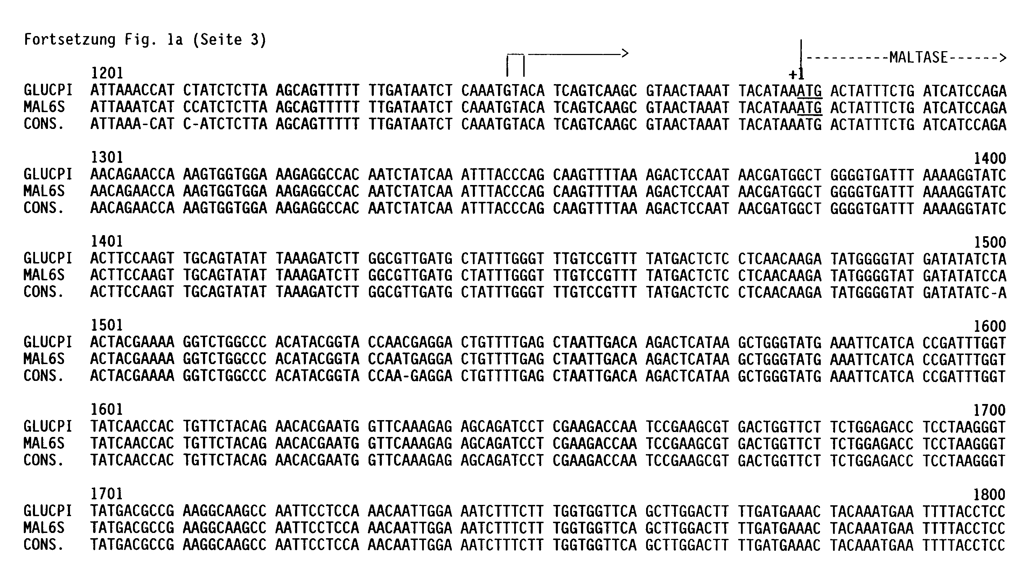

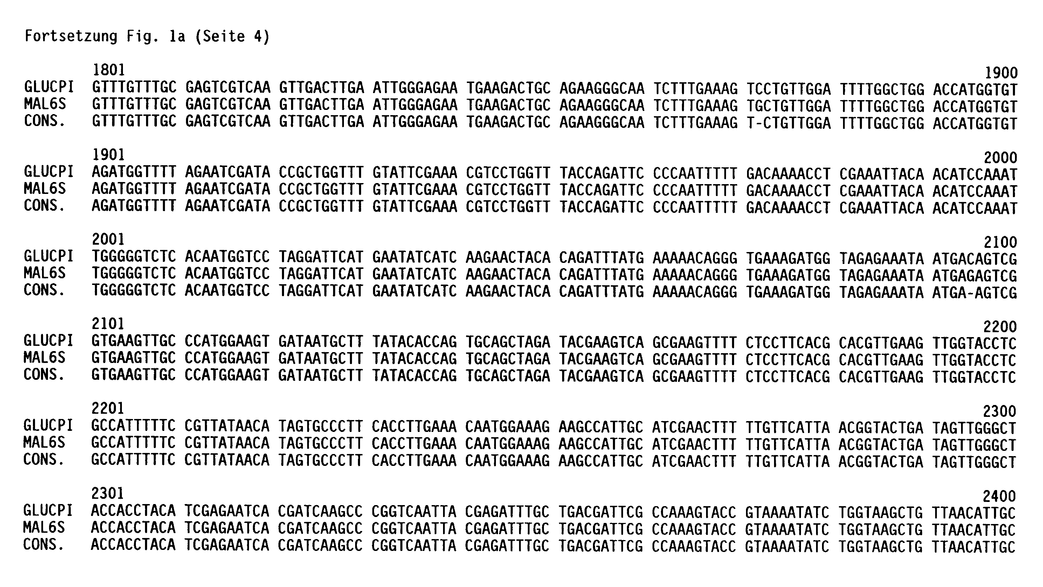

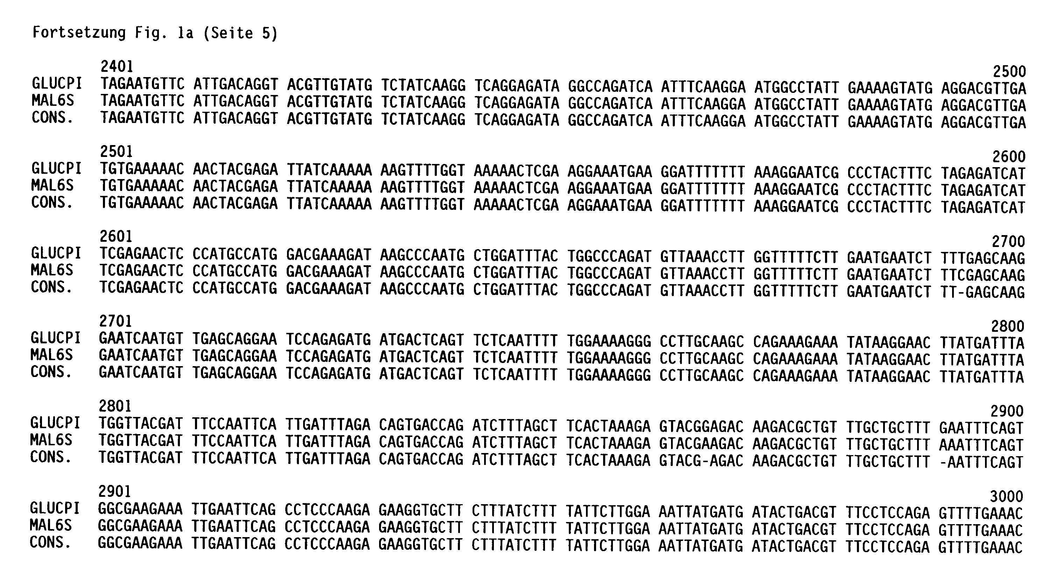

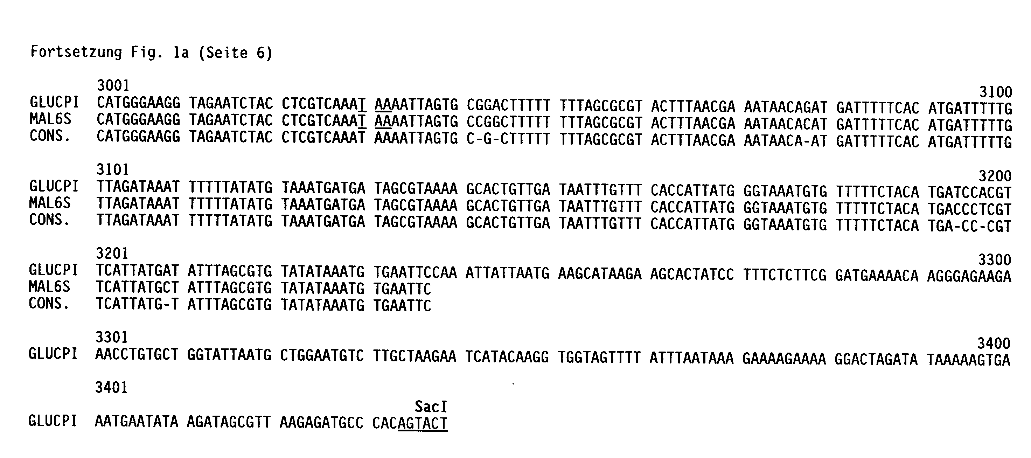

Es wurde nämlich überraschenderweise gefunden, daß die Expression von homologen und heterologen Proteinen oder proteinhaltigen Genprodukten, wie beispielsweise von posttranslational modifizierten, nämlich z.B. glykosilierten, acetylierten, phosphorylierten Proteinen, verbessert werden kann, wenn anstelle des vollständigen α-Glucosidase pI-Promotors ein verkürzter α-Glucosidase pI-Promotor benutzt wird. Als Bezugspunkt +1 für die Numerierung der Promotorsequenz dient hierbei das Nukleotid A des ATG-Startcodons des α-Glucosidase-pI-Gens. Der Ausdruck "upstream" bezeichnet Sequenzen, die in Richtung des 5'-Endes der DNA-Sequenz relativ zu den genannten Bereichen liegen. Die Sequenz des α-Glucosidase pI-Gens ist in der Fig. 1a, jeweils oberste Zeile, im Vergleich mit dem MAL6S-Gen (MAL6S-Maltase), jeweils mittlere Zeile, dargestellt. In einer bevorzugten Ausführungsform der Erfindung ist der Bereich upstream -427, besonders bevorzugt der Bereich upstream der SspI-Schnittstelle bei -386 der α-Glucosidase-pI-Promotorregion deletiert. Hierbei kann der verbliebene Teil der Promotorsequenz upstream des Nukleotids +1 gegebenenfalls weitere Teil-oder Einzelnukleotid-Veränderungen wie z.B. Substitution, Deletionen und Additionen aufweisen. Um jedoch eine maximale Aktivität des α-Glucosidase-Promotors zu erhalten, wird bevorzugt der Bereich zwischen den Nukleotiden -103 und -109 nicht verändert (100% Homologie) und im Bereich zwischen den Nukleotiden -23 und -43 sollte die Sequenz mindestens zu 95% homolog zu der in Fig. 1a gezeigten Sequenz sein.

Insgesamt ist es besonders bevorzugt, wenn der Bereich zwischen den Nukleotiden +1 bis -140 zu mindestens 90% homolog zur entsprechenden Sequenz in Fig. 1a ist.

Unter einem erfindungsgemäßen Expressionsvektor ist nicht notwendigerweise ein DNA-Molekül zu verstehen, das einen Replikationsursprung besitzt und sich eigenständig vermehren kann, sondern es kann sich auch um ein integrierendes DNA-Fragment handeln, das durch Rekombination in die Wirtszell-DNA eingebracht wird und dessen Transkription gemeinsam mit der der Wirts-DNA stattfindet.

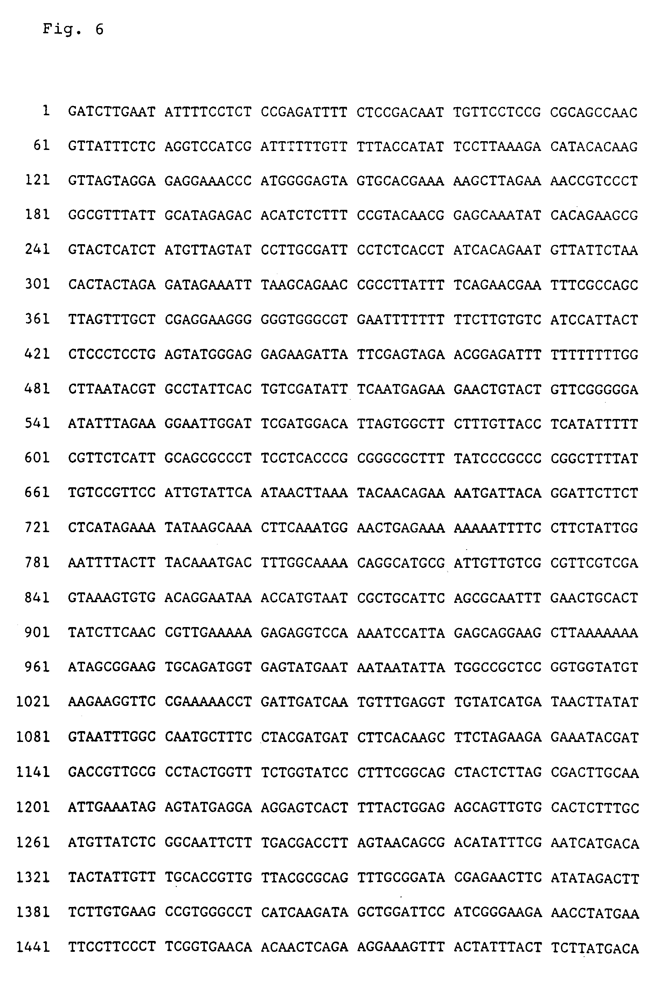

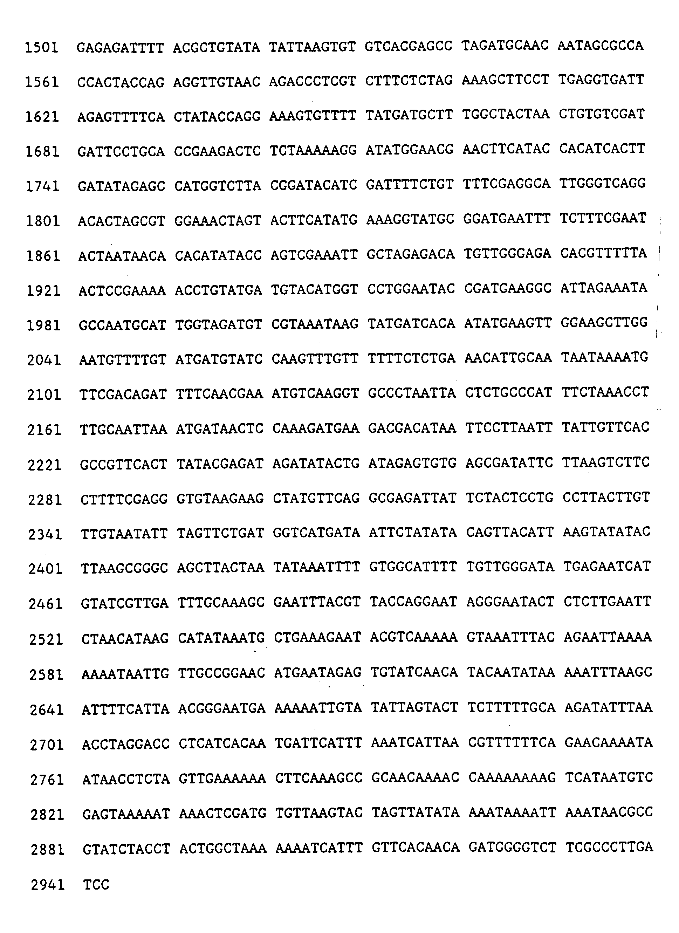

In einer weiteren bevorzugten Ausführungsform enthält der Expressionsvektor zusätzlich ein oder mehrere positive Regulatorgene. Hierdurch kann die Promotoraktivität der verkürzten DNA-Sequenz des α-Glucosidase pI-Promotors erhöht werden. Bevorzugt wird ein positives Regulatorgen aus einem MAL-Locus verwendet. Derartige MALp-Gene sind beispielsweise in Genetika (1976) 12, 87-100, beschrieben. Bevorzugt wird ein mutiertes positives Regulatorgen eingefügt, welches einen konstitutiven, aber noch Glucose reprimierbaren α-Glucosidase-Promotor erzeugt. Ein derartiges mutiertes Regulatorgen wird gewonnen durch die Isolierung von Revertanten nach Mutagenisierung von malp-Regulationsmutanten, d.h. von Mutanten, die wieder in der Lage sind, mit Maltose als einziger C-Quelle zu wachsen. Der gewünschte Phenotyp der Revertanten (konstitutive, aber noch Glucose-reprimierbare Maltase) wird nach Glucoseanzucht bestimmt. Anschließend wird das mutierte positive Regulatorgen nach bekannten Methoden kloniert und dann in den Expressionsvektor eingefügt. Besonders bevorzugt wird als positives Regulatorgen das MAL2-8$^c$p-Gen (Fig. 6) in den Expressionsvektor eingefügt. Dieses Gen kodiert für ein Mutantenallel des klassischen MAL2p-Gens. Es verursacht eine konstitutive aber weiterhin Glucose-reprimierbare Genexpression (MGG 134 (1974) 261-272; Current Genetics 9 (1985) 539-545). In einer weiteren bevorzugten Ausführungsform enthält der erfindungsgemäße Expressionsvektor zusätzlich eine Restriktionsschnittstelle, die das Einsetzen eines Fremdgens an einer Stelle ermöglicht an der das Fremdgen bei der Expression der Kontrolle des verkürzten α-Glucosidase pI-Promotors unterliegt. In einer weiteren bevorzugten Ausführungsform enthält der erfindungsgemäße Expressionsvektor zum Einsetzen eines Fremdgens eine Polylinkerregion, d.h. eine synthetische DNA-Region, die eine Vielzahl von Restriktionsschnittstellen enthält, wodurch das Einsetzen von Fremdgenen erheblich erleichtert wird. Auch diese Polylinkerregion ist dabei in einer Position im Expressionsvektor vorhanden, daß ein darin insertiertes Fremdgen der Kontrolle des verkürzten α-Glucosidase pI-Promotors unterliegt.

Für die weiteren allgemeinen, bekannten Anforderungen an einen eukaryontischen Expressionsvektor wird auf E.L. Winnacker, Gene und Klone, 1985, Verlag Chemie, Kapitel 5 und 8 verwiesen.

Als eukaryontische DNA, in die im erfindungsgemäßen Expressionsvektor der regulierbare Promotor insertiert ist, sind alle für die jeweilige eukaryontische Wirtszelle verwendbaren DNA-Moleküle geeignet, z.B. Plasmide, Viren, integrierende lineare DNA-Moleküle, künstliche Chromosomen etc. Bevorzugt ist die DNA ein Plasmid oder ein integriertes DNA-Fragment. Besonders bevorzugt ist die DNA hierbei ein Plasmid, das in der Zelle in hoher Kopienzahl vorkommt. Wird die Expression des erfindungsgemäßen Expressionsvektors in Hefezellen durchgeführt, wird als Vektor bevorzugt ein in hoher Kopienzahl in der Zelle vorkommender Hefevektor verwendet. Beispielhaft für solche Vektoren sind die YEp- und YRp-Vektoren.

Besonders bevorzugt sind hierbei YEp/S3, YEp/S4 und YEp/5C6b3. Die Herstellung dieser besonders bevorzugten Vektoren ist in den Beispielen 3 und 4 beschrieben.

Ebenfalls besonders bevorzugt sind weitere erfindungsgemäße Expressionsvektoren, die als zu exprimierendes Gen das Gen für die alpha-Glucosidase PI enthalten.

Ein weiterer Gegenstand der Erfindung ist ein Verfahren zur regulierbaren Herstellung von homologen und heterologen Proteinen oder proteinhaltigen Genprodukten in eukaryontischen Wirtszellen, bei dem man in einen der erfindungsgemäßen eukaryontischen Expressionsvektoren eine für das gewünschte Protein oder Genprodukt kodierende DNA-Sequenz insertiert, den erhaltenen Vektor durch Transformation in eine geeignete Wirtszelle einbringt, die Zellen kultiviert und das gebildete Protein oder Genprodukt aus den Zellen oder ihrem Nährmedium isoliert.

Durch die Verwendung eines erfindungsgemäßen eukaryontischen Expressionsvektors zur Herstellung von homologen und heterologen Proteinen oder proteinhaltigen Genprodukten wird überraschenderweise eine bedeutend höhere Ausbeute der Proteine erhalten als bei Verwendung von Expressionsvektoren, die die gesamte α-Glucosidase pI-Promotorregion enthalten. Die Expression des gewünschten Proteins wird im erfindungsgemäßen Verfahren bevorzugt durch Glucose reprimiert und durch Maltose induziert. Hierdurch besteht die Möglichkeit, die maximale Expression des gewünschten Proteins erst nach Bildung einer ausreichenden Biomasse von Wirtszellen zu induzieren, und dadurch eine höhere Ausbeute zu erhalten.

Für die bekannten Methoden des Insertierens einer DNA-Sequenz in einen eukaryontischen Vektor und die Transformation von eukaryontischen Zellen wird wiederum beispielsweise auf E.-L. Winnacker, Gene und Klone, 1985, Verlag Chemie, insbesondere Kapitel 3, 5 und 8 und die dort zitierte Originalliteratur verwiesen.

In einer besonders bevorzugten Ausführungsform verwendet man als Wirtszellen Hefezellen. Es ist hierbei besonders bevorzugt, einen Expressionsvektor zu verwenden, der als eukaryontische DNA ein in der Zelle in hoher Kopie vorkommendes Hefeplasmid aufweist. Beispiele für solche bevorzugten Expressionsvektoren sind YEp/S3 und YEp/S4. In einer weiteren bevorzugten Ausführungsform verwendet man einen Expressionsvektor, der zusätzlich zur Promotorsequenz ein positives Regulatorgen, besonders bevorzugt das MAL2-8$^c$p-Gen (Fig. 6) enthält. Ein solcher Expressionsvektor ist beispielsweise YEp/5C6b3.

In einer weiteren bevorzugten Ausführungsform bringt man zusätzlich zu dem Expressionsvektor in die Zelle ein Plasmid oder integrierendes DNA-Fragment ein, das ein positives Regulatorgen enthält, das den α-Glucosidase pI-Promotor stimuliert. Ein solches Regulatorgen ist wiederum bevorzugt das MAL2-8$^c$p-Gen.

In noch einer weiteren bevorzugten Ausführungsform verwendet man eine Wirtszelle, die selbst ein positives Regulatorgen (z.B. MALp-Gen) enthält, wobei es wiederum besonders bevorzugt ist, wenn dieses Regulatorgen in der Wirtszelle überexprimiert ist.

In einer weiteren bevorzugten Ausführungsform der Erfindung insertiert man die DNA-Sequenz für α-Glucosidase PI in einen erfindungsgemäßen Expressionsvektor und isoliert α-Glucosidase PI als gewünschtes Produkt des erfindungsgemäßen Verfahrens.

Erfindungsgemäß gelingt es also, Expressionsvektoren bereitzustellen, die auf einfache und kostengünstige Weise reguliert werden können und in die für ein gewünschtes Genprodukt kodierende Fremdgene insertiert werden können. Weiter gelingt es, ein Verfahren bereitzustellen, das es unter Verwendung dieser Expressionsvektoren ermöglicht, homologe und heterologe Proteine oder proteinhaltige Genprodukte in eukaryontischen Wirtszellen, vor allem in Hefe, herzustellen, wobei der Zeitpunkt der maximalen Expression in der Wachstumsphase der Wirtszellen durch einfache aber effektive Regulation bestimmt werden kann.

Die folgenden Beispiele in Verbindung mit den Figuren erläutern die Erfindung weiter.

- Fig. 1a zeigt die DNA-Sequenz des α-Glucosidase pI-Gens im Vergleich zum MAL6S Maltasegen;
- Fig. 1b zeigt die Promotor-Organisation von GLUCPI im Vergleich mit MAL6S;
- Fig. 2 zeigt schematisch das Plasmid YRp/GLUCPI;
- Fig. 3A-F zeigt schematisch die in den Beispielen hergestellten Vektorkonstruktionen;
- Fig. 4 zeigt eine Derepressionskurve von ABYSMAL81 Transformanten unter Kontrolle des authentischen (A), verkürzten (B) und verkürzten MAL2-8$^c$p stimulierten (C) α-Glucosidase-Promotors in Glucose Minimal-Medium und
- Fig. 5 zeigt eine SDS-Polyacrylamidgelelektrophorese-Analyse des α-Glucosidase pI-env-Fusionsproteins, welches in dem Wirtsstamm ABYSMAL81 exprimiert wurde als Coomassie-Färbung (A) und Immunoblotanalyse (B).
- Fig. 6 zeigt die DNA-Sequenz des MAL2-8$^c$p-Gens.

## Beispiele

α-Glucosidase pI Promotor aus S. cerevisiae

Zur Manipulation von DNA wurden Standardmethoden benutzt, wie sie bei Maniatis et al. (1982) in Molecular Cloning, Cold Spring Harbor Laboratory, Cold Spring Harbor, New York, beschrieben sind. Die verwendeten molekularbiologischen Reagenzien wurden nach Angaben des Herstellers eingesetzt.

S. cerevisiae wurde gemäß der Transformationsmethode von Beggs (Nature 275 (1978) 104-109) transformiert.

Bestimmung der spezifischen α-Glucosidaseaktivität

Die Hefetransformanten und Bäckerhefe wurden in Minimalmedium (YNB (yeast nitrogen base, Salz-Vitamin-Gemisch, Difco) 0,67%, CAA (Casaminosäuren, Proteinhydrolysat, Difco) 0,5%, Adenin 30 mg/1) und einer gewünschten C-Quelle (2 bis 3%) angezogen. In der logarithmischen bis spätlogarithmischen Wachstumsphase wurden die Zellen aus 5 ml Kultur geerntet, einmal mit 10 mmol/1 Phosphatpuffer, pH 6,8 gewaschen, durch Homogenisierung mit einem Whirlmix aufgeschlossen und die Zelltrümmer durch Zentrifugation abgetrennt (MGG 145 (1976) 327-333).

Die Bestimmung der spezifischen α-Glucosidaseaktivität erfolgte anhand der Hydrolyse von p-Nitrophenyl-α-D-Glucopyranosid (MGG 151 (1977) 95-103) und der Proteinbestimmung nach Zamenhof (Methods in Enzymol. 3, (1957) 702).

**Beispiel 1**

Klonierung des α-Glucosidase pI Promotors aus S. cerevisiae und Sequenzvergleich mit dem homologen MAL6S-Promotor aus S. carlsbergensis.

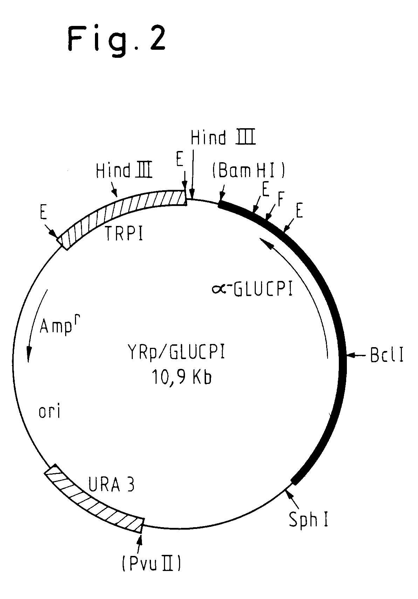

Das α-Glucosidase pI-Gen aus S. cerevisiae wurde durch Komplementation einer auxotrophen Maltasestrukturgenmutante TCY70 (α mallS-Δ ura3-52) (MGG 200 (1985) 1-8), DSM 4315, aus einem Bäckerhefegenpool nach bekannten Methoden isoliert. Das α-Glucosidase pI-Gen wurde auf einem DNA-Fragment von ca. 3,5 kBp Länge in die BamHI-Schnittstelle des zur Klonierung verwendeten Hefe-/E.coli-Shuttlevektors YRU7 integriert. YRU7 ist aus dem bekannten Hefevektor YRp7 (PNAS 76 (1979) 1035-1039; Nature 282 - (1979) 39-43, Cold Spring Harbor Symp. Quant. Biol. 43 (1979) 77-90, Gene 10, (1980) 157-166) durch Insertion des URA3-Hefemarkers (HindIII-Fragment, 1,1 kBp nach Auffüllung der überhängenden 5′-Enden mit Klenow-Polymerase) aus dem Hefevektor YEp24 (Gene 8 (1979) 17-24, Cold Spring Harbor Symp. Quant. Biol. 43 (1979) 77-90, Gene 29 (1984) 113-124, Nature 286 (1980) 860-865) in die PvuII-Schnittstelle von YRp7 hervorgegangen. Dieser um das α-Glucosidase pI-Gen erweiterte Vektor YRp/GLUCPI wurde hinterlegt, DSM 4173 P und ist in Fig. 2 dargestellt. Das α-Glucosidase pI-Gen wurde nach der Didesoxykettenabbruchreaktion (PNAS 74 (1977) 5463-5467; J. Mol. Biol. 143 (1980) 161-178) sequenziert und mit der Sequenz des MAL6S-Maltasegens (Gene 41 (1986) 75-84) aus S. carlsbergensis verglichen (Fig. 1a).

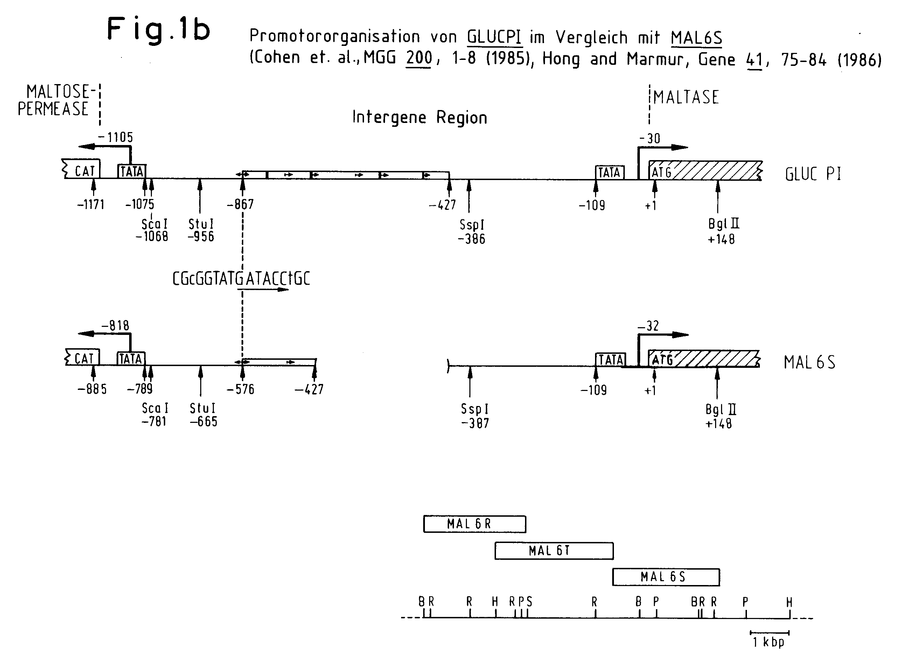

Der Maltase- bzw. α-Glucosidase pI-Promotor ist ein in 2 Richtungen aktiver Promotor (divergenter Promotor) der in Anwesenheit von Maltose die koordinierte Synthese von Maltase und Maltosepermease bewirkt. Der divergente MAL6S- und der α-GLUCPI-Promotor sind bezüglich der TATA-Boxen, mRNA-Startpunkte und Translationsstarts für Maltase und Maltosepermease identisch. Jedoch besteht der divergente MAL6S-Promotor nur aus ca. 800 Bp im Gegensatz zu den ca. 1,2 kBp des α-Glucosidase pI-Promotors. Der Hauptunterschied besteht in der dreifachen Wiederholung eines DNA-Abschnitts von jeweils 147 Bp ("Triplett-repeat", Position -427 bis -867, Bezugspunkt ATG α-Glucosidase pI +1). Im homologen Promotorbereich werden zusätzlich noch 33 DNA-Sequenzunterschiede gefunden. Die Terminatorregion weist nur sehr wenig Sequenzunterschiede auf (Fig. 1a und 1b).

**Beispiel 2**

Herstellung eines α-Glucosidase pI-Expressionsvektors mit vollständiger α-Glucosidase-pI-Expressionskassette

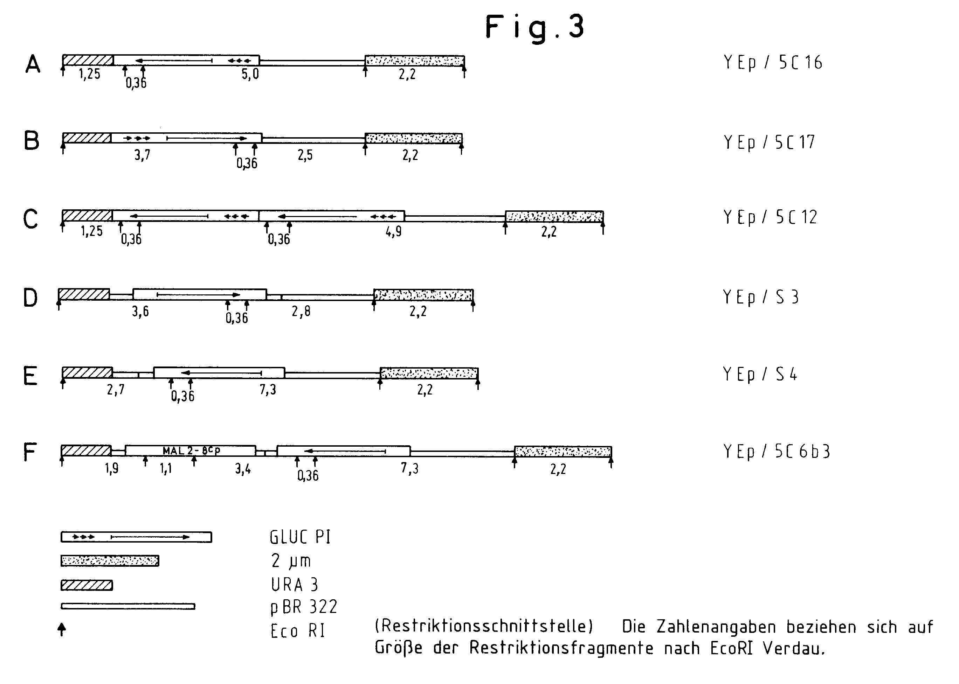

Das Plasmid YRp/GLUCPI, DSM 4173 P, das die komplette α-Glucosidase-pI-Expressionskassette bestehend aus divergentem Promotor mit "upstream activation site", Strukturgen und Terminator enthält, wurde mit der Restriktionsendonuklease ScaI verdaut, das ca. 3,3 kBp lange ScaI-Fragment isoliert und in das isolierte 5,6 kBp lange PvuII/SmaI-Vektorfragment aus YEp24 (Gene 8 (1979) 17-24) ligiert. Die entstehenden Plasmide YEp/5C16 und YEp/5C17 (Fig. 3A und 3B) enthalten die α-Glucosidase-pI-Expressionskassette in gleicher bzw. gegenläufiger Orientierung zum β-Lactamasegen. Die Konstruktion YEp/5C12 (Fig. 3C) enthält 2 α-Glucosidase pI Kassetten nacheinandergeschaltet (in "tandem") in gleicher Orientierung zum β-Lactamasegen. Diese Vektoren sind dadurch charakterisiert, daß sie die 1068 Bp lange Promotorregion, das Strukturgen und den Terminator der α-Glucosidase pI enthalten und der Maltosepermeasepromotor einschließlich der dazugehörigen TATA-Box entfernt wurde (Fig. 1b).

Transformanten der Hefestämme TCY70, DSM 4315, (α mallS-Δ ura3-52) (MGG 200 (1985) 1-8) und ABYSMAL81, (Herstellung siehe Beispiel 7) (ura3-52 mallS-Δ lys pra1 prb1 prc1 cps1), die diese α-Glucosidase pI Expressionsplasmide ("Promotor mit Triplett-Repeat") enthalten, erreichen auf Minimalmedium (YNB 0,67%, CAA 0,5%, Adenin 30 mg/l) unter reprimierenden Bedingungen mit 2% Glucose in der logarithmischen bis spätlogarithmischen Wachstumsphase nur Expressionswerte (spezifische α-Glucosidase-Aktivität) von ca. 0,2 U/mg. Nach Maltoseinduktion (Überführung in Minimalmedium mit 2% Maltose) wurden Expressionswerte von ca. 1 U/mg erreicht (Tabelle 1). Hinsichtlich der α-Glucosidaseexpression unterscheiden sich diese Transformanten trotz erhöhter Gendosis (2 Kopien in "tandem", "multicopy"-Plasmid) kaum von Wildtypstämmen (Bäckerhefe).

**Beispiel 3**

Herstellung eines Vektors mit verkürztem α-Glucosidase PI-Promotor

Der im Beispiel 1 beschriebene Vektor YRp/GLUCPI wurde mit den Restriktionsendonukleasen SspI und HindIII verdaut, das ca. 3,0 kBp lange SspI/HindIII-Fragment isoliert und in das isolierte PvuII/SphI-Vektorfragment aus YEp24 nach Auffüllung des überhängenden 5'-Endes der HindIII- und Abbau des überhängenden 3'-Endes der SphI-Restriktionsschnittstelle mit Klenow-Polymerase ligiert. Die entstehenden Plasmide YEp/S3 und YEp/S4 (Fig. 3D und 3E) enthalten die α-Glucosidase pI-Expressionskassette in gleichläufiger bzw. gegenläufiger Orientierung zum β-Lactamasegen. Sie sind dadurch charakterisiert, daß der α-Glucosidase Promotor an der Position -386 (SspI-Schnittstelle, Bezugspunkt ATG α-Glucosidase +1) beginnt und somit der "Triplett-repeat" deletiert ist.

Transformanten der Hefestämme TCY70, DSM 4315, und ABYSMAL81 (Beispiel 7) die diese α-Glucosidase pI Expressionsplasmide mit verkürztem Promotor enthalten, sind durch Glucose reprimierbar (Minimalmedium mit 2% Glucose) und erreichen auf Minimalmedium mit 2% Maltose ihr Induktionsmaximum. Bei gegenläufiger Orientierung zum β-Lactamasegen werden 1,5- bis 2fach höhere spezifische Expressionswerte erreicht (siehe Tabelle 1).

**Beispiel 4**

Herstellung von α-Glucosidase pI-Expressionsplasmiden mit verkürzten α-Glucosidase pI-Promotor in Kombination mit dem Maltaseregulatorgen MAL2-8$^c$p

Das von Zimmermann und Mitarbeitern isolierte (MGG 134 (1974) 261-272) und kürzlich klonierte MAL2-8$^c$p Mutantenallel (Curr. Gen. 9 (1985) 539-545, Sequenz siehe Fig. 6) bewirkt eine konstitutive, aber weiterhin durch Glucose reprimierbare Synthese der MAL2S-Maltase.

Das Plasmid pRM2, DSM 4314P, welches dieses Mutantenallel enthält, (Curr. Gen. 9 (1985) 539-545), wird mit der Restriktionsendonuklease SalI verdaut, die überhängenden 5'-Enden mit Klenow-Polymerase aufgefüllt, mit BamH1-Linkern (d(CGGGATCCCG)) versehen, mit BamH1 nachgespalten, das ca. 3,1 kBp lange MAL2-8$^c$p-Gen enthaltende BamH1-Fragment isoliert und in den in Beispiel 3 beschriebenen mit BamHI-verdauten Vektor YEp/S4 ligiert. Die Vektorkonstruktion wurde mit YEp/5C6b3 bezeichnet (Fig. 3F).

Die Anwesenheit des MAL2-8$^c$p-Gens auf Plasmiden mit verkürztem α-Glucosidase pI-Promotor beeinflußt das Wachstum der Transformanten nicht und führt auf allen verwendeten Medien (siehe Beispiel 2) zu 1,5- bis 2fach höheren Expressionswerten (Tabelle 1). Alternativ kann das MAL2-8$^c$p-Gen durch Cotransformation in die Hefe eingeschleust werden und so seine Effekte hervorbringen.

**Beispiel 5**

Glucoserepression und Derepression von plasmidcodierten α-Glucosidase nach Verbrauch des reprimierenden Substrats Glucose.

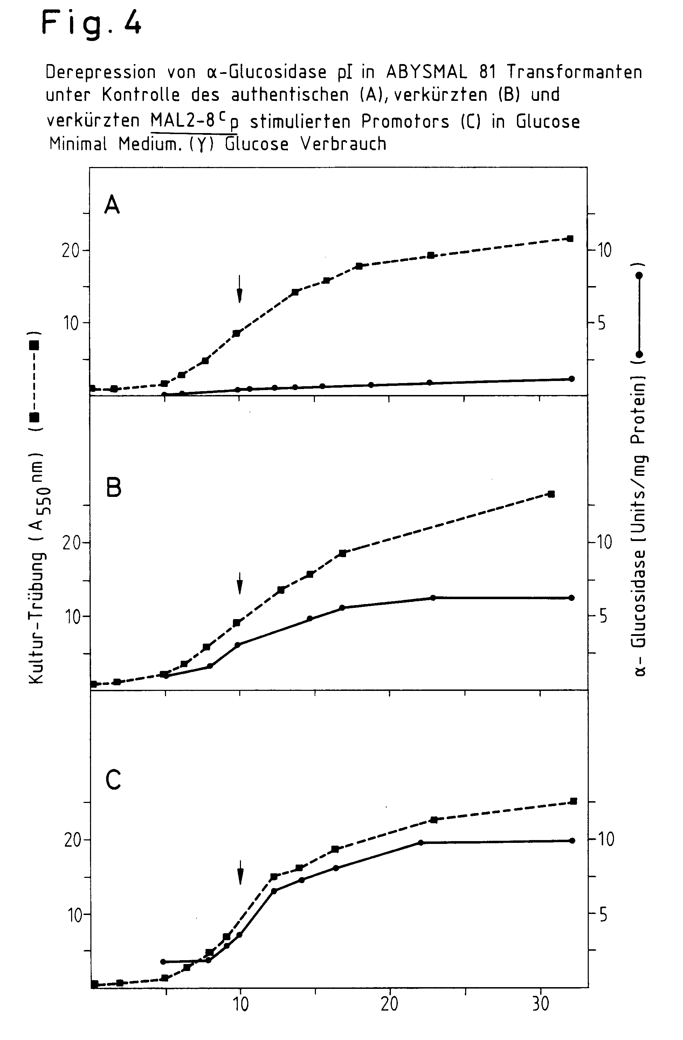

Die Derepression der α-Glucosidase pI nach Glucoseverbrauch in Minimalmedien in ABYSMAL81-Transformanten wurde beispielhaft für die unterschiedlichen α-Glucosidaseexpressionskassetten YEp/5C16, YEp/S4 und YEp/5C6b3 durch Aufnahme der Derepressionskinetiken (Fig. 4) bestimmt. Gemessen wurde das Zellwachstum anhand der Trübung bei 550 nm und die spez. α-Glucosidaseaktivität über einen Zeitraum von ca. 30 Stunden. Ebenfalls wurde der Zeitpunkt des Glucoseverbrauchs ermittelt.

Unabhängig von der α-Glucosidase pI-Promotorkonstruktion und der Anwesenheit von MAL2-8$^c$p steigt die spez. α-Glucosidase pI-Aktivität nach Glucoseverbrauch um einen Faktor von 5 bis 10 innerhalb von 20 Stunden (Fig. 4). Die Verkürzung des Promotors oder/und die Anwesenheit des MAL2-8$^c$p-Gens moduliert die maximal zu erreichende spez. α-Glucosidase pI-Aktivität.

**Beispiel 6**

Heterologe Expression eines Fusionsproteins, bestehend aus dem N-terminalen Teil der α-Glucosidase und HIV1-Antigen unter Verwendung der α-Glucosidase pI Expressionskassette.

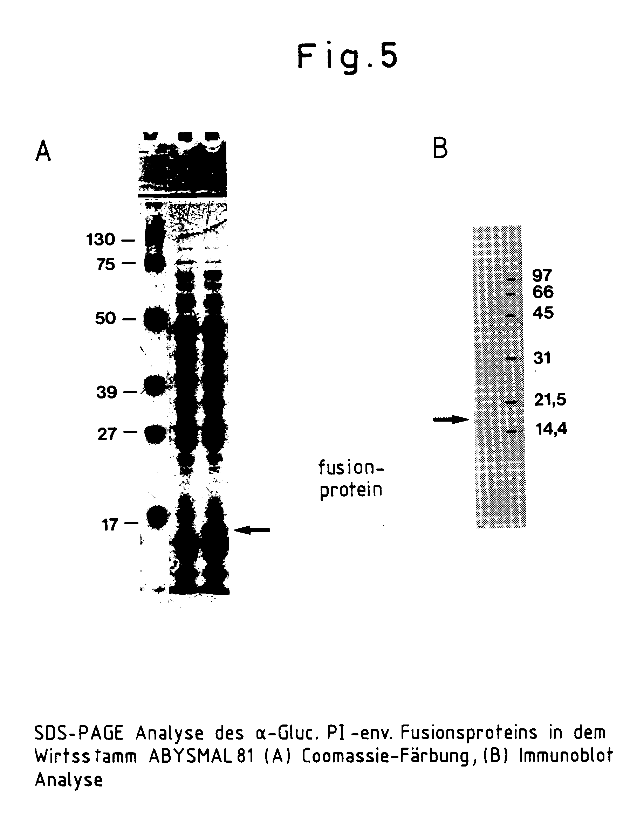

In dem α-Glucosidase PI-Expressionsvektor YEp/5C6b3 (Beispiel 4) wurde das 1,4 KBp lange BglII-Fragment, das für ca. 80% der α-Glucosidase PI codiert, gegen ein ca. 300 Bp langes DNA-Fragment, das

für einen Teil des gp41-Membranproteins des HIV1-Retrovirus codiert, ausgetauscht. Dazu wurde ein ca. 300 Bp lange RsaI/HindIII-Fragment (Sequenz vgl. Sequenz von WMJ-1 von Pos. 1638 bis Pos. 1943 aus Fig. 1 von Cell 45 (1986) 637-648) in den mit HinCII und HindIII verdauten E.coli-Vektor pUC18 (M13mp18 und pUC19 Sequenz in Gene 33 (1985) 103-119) subcloniert (Konstruktion: pUC18 HRH.300). Aus pUC18 HRH.300 wurde das ca. 320 Bp lange BamH1/HindIII-Fragment isoliert und in das ca. 5,2 Bp lange pUR278 BamH1/HindIII-Vektorfragment ligiert (Sequenz in EMBO 2 (1983) 1791-1794) (Konstruktion: pUR278HRH.300). Das Plasmid pUR278HRH.300 wurde mit HindIII verdaut, die überhängenden 5'-Enden mit "Klenow Polymerase" aufgefüllt und mit BamHI-Linkern der Sequenz d(GGGATCCC) versehen. Danach wurde mit BamHI nachgespalten, das ca. 300 Bp lange BamHI-Fragment isoliert und in das ca. 11 KBp lange YEp/5C6b3-BglII-Vektorfragment ligiert. Bei richtiger Orientierung der gp41-Membranpolypeptid-DNA entsteht ein Fusionsprotein, bestehend aus dem N-Terminus der α-Glucosidase (50 Aminosäuren), 4 konstruktionsbedingten Aminosäuren an der Fusionsstelle, 101 Aminosäuren des gp41-Membranproteins und 3 konstruktionsbedingten Aminosäuren am C-Terminus mit einem Molekulargewicht von ca. 18500 D. Die gewünschte Konstruktion wurde über das exprimierte Fusionsprotein in dem Protease-defizienten Hefeexpressionsstamm ABYSMAL81, Beispiel 7, nach Transformation und Anzucht (vgl. unten) gefolgt von SDS-Gelelektrophorese und Westernblot anhand von Immunreaktivität mit humanen HIV1-Seren isoliert. Das Fusionsprotein wurde zu ca. 5% des Gesamtproteins exprimiert und war als dominante Bande in SDS-Polyacrylamidgelen nach Coomassie-Anfärbung sichtbar (Fig. 5).

Zur Expression des α-Gluc.PI-gp41-Fusionsproteins wurden die Transformanten auf Selektivmedium (0,67% YNB, 0,5% CAA, 30 mg/l Adenin) mit 2% Glucose und 2% Maltose angezogen. Nach einer Induktionsphase von 10 bis 20 Stunden (nach Glucoseverbrauch) wurden die Zellen geerntet.

**Beispiel 7**

Zur Herstellung des Saccharomycesstammes ABYSMAL81 wurde der haploide Saccharomyces cerevisiae-Stamm ABYSD-11 (a pra1 prb1 prc1 prd1, cps1, ade lys his7), DSM 4322, der bzgl. der Proteasen A, B und D und der Carboxypeptidasen Y und S defizient ist, und zusätzlich eine Auxotrophie in der Adenin-, Histidin- und Lysin-Biosynthese besitzt, mit dem Saccharomyces carlsbergensisStamm, TCY2824-1A, (α malls-Δ ura3-52 his4), DSM 4317, gekreuzt, der durch ein defektes α-Glucosidase-Strukturgen und durch eine Auxotrophie in der Uracil- und Histidin-Biosynthese charakterisiert ist.

Anschließend wurde eine Sporulation durchgeführt und die entstandenen Hefe-Segreganten auf ihre Auxotrophien durch Ausplattieren auf verschiedenen Selektionsmedien und auf ihre Protease-Defizienzen durch Bestimmung der Protease-Aktivitäten in Zellysaten überprüft. Der Stamm ABYSMAL81 (ura3-52 mallS-Δ lys pra1 prb1 prc1 cps1) wurde identifiziert durch:

Nachweis der Protease-Defizienzen

Die Segreganten wurden in 5 ml YEPD-Medium (1% Hefeextrakt, 2% Pepton, 2% Glucose) angezogen, die Zellen in der spät-logarithmischen bis früh-stationären Wachstumsphase geerntet, zweimal mit Wasser gewaschen und mit Glasperlen durch Homogenisieren auf einem Whirlmix aufgeschlossen (MGG 145 (1976) 327-333). Die Zellen wurden mit 1 ml 20 mmol/l Tris (HCl), pH 7,0 extrahiert und der Überstand nach Zentrifugation als Rohextrakt weiterverarbeitet. Zur Aktivierung der Proteasen wurde der Rohextrakt auf pH 5,0 titriert und 24 Stunden bei 25 °C inkubiert.

Nachweis der Protease A-Defizienz (pra1)

Keine Hydrolyse durch Zellextrakte von 1,2% säuredenaturiertem Hämoglobin, pH 3,0 (Eur. J. Biochem. 42 (1974) 621-626).

0,5 ml 0,1 mol/l Lactat-Puffer, pH 3,0 mit 1,2% säuredenaturiertem Hämoglobin wurden mit 0,1 ml Zellysat bei 25 °C inkubiert. Nach 30 Minuten wurde die Reaktion mit 0,5 ml l0%iger Trichloressigsäure gestoppt und nach Zentrifugation die Trichloressigsäure löslichen Produkte entweder durch Absorptionsmessung bei 280 nm oder durch eine modifizierte Folinbestimmung nach McDonald und Chen (Anal. Biochem. 10 (1965) 175-177) bestimmt.

Zur Berechnung der spezifischen Aktivität wurde eine Proteinbestimmung nach Zamenhof durchgeführt (Methods Enzymol. 3 (1957) 702).

Eine Protease A-Defizienz liegt vor, wenn die spezifische hydrolytische Aktivität von Zellysatien gegenüber säuredenaturiertem Hämoglobin auf kleiner 5% im Vergleich mit einem Wildtypstamm herabgesetzt ist.

Nachweis der Protease B-Defizienz (prb1)

Keine Hydrolyse durch Zellextrakte von 2,4% Azocoll bei pH 7 (Eur. J. Biochem. 42 (1974) 621-626).

0,5 ml einer 2,4%igen Azocoll Suspension in 0,1 mol/l Phosphatpuffer, pH 7,0 wurden mit 0,1 ml Zellysat unter Schütteln bei 25 °C inkubiert. Zur Aktivierung der Protease B wurde der Rohextrakt vor der Aktivitätsbestimmung mit Natriumdodecylsulfat (Endkonzentration 0,25%) versetzt.

Nach 30 Minuten wurde die Reaktion durch Zugabe von 0,5 ml 10%iger Trichloressigsäure gestoppt und nach Zentrifugation die Extinktion im Überstand bei 550 nm bestimmt.

Eine Protease B-Defizienz liegt vor, wenn die spezifische hydrolytische Aktivität von Zellysaten gegenüber Azocoll auf kleiner 5% im Vergleich mit einem Wildtypstamm herabgesetzt ist.

Nachweis der Protease D-Defizienz (prd1)

Keine Hydrolyse durch Zellextrakte von 0,5 mmol/l Bz-Pro-Phe-Arg-NA (Benzoyl-L-prolyl-L-phenylalanyl-L-arginyl-p-nitroanilid) in 50 mmol/l Tris-Maleat-Puffer, pH 7,0 in Gegenwart von Aminopeptidase M (J. Biol. Chem. 260 (1985) 4585-4590).

Testprinzip:

Bz-Pro-Phe-Arg-NA —Protease D→ Bz-Pro + Phe-Arg-NA

Phe-Arg-NA —Aminopeptidase M→ Phe+Arg+4-Nitroanilin

0,03 ml 0,5 mol/l Tris-Maleat Puffer, pH 7,0 wurden mit 0,015 ml 10 mmol/l Bz-Pro-Phe-Arg-NA (gelöst in Dimethylsulfoxid), 10 µl (40 µg, 240 mU) Aminopeptidase M, 0,145 ml Wasser und 0,1 ml Rohextrakt gemischt und die Extinktionsänderung bei 405 nm gegen einen Reagenzienleerwert bestimmt.

Eine Protease D-Defizienz liegt vor, wenn die spezifische hydrolytische Aktivität gegenüber Bz-Pro-Phe-Arg-NA auf kleiner 10% im Vergleich mit einem Wildtypstamm herabgesetzt ist.

Nachweis der Carboxypeptidase Y-Defizienz (prc1)

Keine Hydrolyse durch Zellextrakte von 0,5 mmol/l Benzoyl-L-Tyrosin-4-nitroanilid, pH 7 (Agr. Biol. Chem. 35 (1971) 658-666).

0,1 ml mit Deoxycholat aktivierter (Endkonzentration 0,5%) Rohextrakt wurden mit 10 ml 0,1 mol/l Phosphatpuffer pH 7,0 und 0,2 ml 3 mmol/l Benzoyl-L-Tyrosin-4-nitroanilid (gelöst in Dimethylformamid) bei 25 °C inkubiert. Nach 10 Minuten wurde die Reaktion mit 1 ml 1 mmol/l Quecksilberchlorid gestoppt und das freigesetzte p-Nitroanilin bei 410 nm bestimmt.

Eine Carboxypeptidase Y-Defizienz liegt vor, wenn die spezifische hydrolytische Aktivität gegenüber Benzoyl-L-Tyrosin-4-nitroanilid auf kleiner 5% im Vergleich mit einem Wildtypstamm herabgesetzt ist.

Nachweis der Carboxypeptidase S-Defizienz (cps1)

Keine Hydrolyse durch Zellextrakte von Cbz-Gly-Leu (Benzyloxycarbonyl-glycyl-L-leucin) bei pH 7,4 mit nachfolgender Analyse des freigesetzten Leucins in einem L-Aminosäure Oxidase-Peroxidase Test (Eur. J. Biochem. 73 (1977) 553-556).

0,5 ml Testlösung (0,25 mg/ml L-Aminosäure Oxidase, 0,4 mg/ml Meerrettich Peroxidase und 0,5 mmol/l $MnCl_2$), 0,4 ml 27,5 mmol/l Cbz-Gly-Leu Lösung (gelöst in 0,2 mol/l Phosphatpuffer, pH 7,0), 0,05 ml o-Dianisidindihydrochlorid (2 mg/ml, gelöst in $H_2O$), 0,05 ml 22 mmol/1 Phenylmethylsulfonylfluorid und 0,1 ml dialysiertes Zellysat (Dialyse: 0,1 mol/l Imidazolchlorid, pH 5,3; 24 Stunden; 25 °C) gemischt und die Extinktionsänderung bei 405 nm bestimmt.

Eine Carboxypeptidase Y-Defizienz liegt vor, wenn die spezifische hydrolytische Aktivität gegenüber Cbz-Gly-Leu auf kleiner 5% im Vergleich mit einem Wildtypstamm herabgesetzt ist.

Anhand der beschriebenen Nachweise wurde festgestellt, daß der Stamm ABYSMAL81 defizient an den Proteasen A, B und den Carboxypeptidasen Y und S ist.

Nachweis der Maltoseverwertungs-Auxotrophie

Kein Wachstum auf synthetischem Komplettmedium I mit 0,67% yeast nitrogen base (YNB, Salz-Vitamingemisch, Difco), 0,5% Casaminosäuren (CAA, Proteinhydrolysat, Difco), 2% Maltose (einzige C-Quelle), 20 mg/l Uracil und 30 mg/l Adenin.

Nachweis der Uracil-Auxotrophie

Kein Wachstum auf synthetischem Komplettmedium II mit 0,67% YNB, 0,5% CAA, 2% Glucose (einzige C-Quelle) und 30 mg/l Adenin.

Nachweis der Lysin-Auxotrophie

Kein Wachstum auf synthetischem Komplettmedium II mit Uracil (20 mg/l) aber ohne Lysin (anstelle von 0,5% CAA wurde ein Aminosäuregemisch ohne Lysin benutzt).

Nachweis der Histidin- und Adenin-Prototrophie

Wachstum auf synthetischem Komplettmedium II mit Uracil (20 mg/l) ohne Adenin und ohne Histidin (anstelle von 0,5% CAA wurde ein Aminosäuregemisch ohne Histidin benutzt).

TABELLE I

| Verhalten von Hefe α-Glucosidase-Transformanten | | | α-Glucosidase Aktivität (mU/mg) | | log. phase Verhältnis |
|---|---|---|---|---|---|
| Stamm | Plasmid | Genotyp/Plasmidkonstruktion | glucose | maltose | (maltose/glucose) |
| 1. TCY70 | - | mallS-Δ ura3-52 | 1 | ---- | ---- |
| 2. ABYSMAL 81 | - | mallS-Δ ura3-52 lys pra1 prb1 prc1 cps1 | 1 | ---- | ---- |
| 3. TCY 70 | YRp/GLUCP1 | 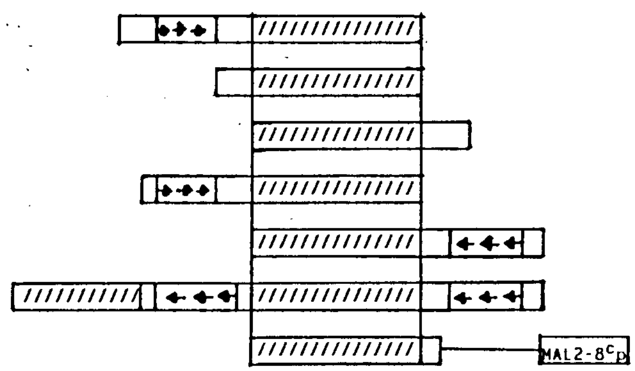 | n.d. | 910 | n.d. |
| 4. TCY 70 | YEp/S3 | | 440 | 2200 | 5 |
| 5. TCY 70 | YEp/S4 | | 800 | 3650 | 4,6 |
| 6. ABYSMAL 81 | YEp/5C16 | | 160 | 1100 | 7,4 |
| 7. ABYSMAL 81 | YEp/5C17 | | 140 | 1000 | 7,1 |
| 8. ABYSMAL 81 | YEp/5C12 | | 180 | 1100 | 6,1 |
| 9. ABYSMAL 81 | YEp/5C6b3 | MAL2-8$^c_p$ | 1820 | 9820 | 5,5 |

## Patentansprüche

1. Eukaryontischer Expressionsvektor, enthaltend einen regulierbaren Promotor insertiert in eine eukaryontische DNA,

**dadurch gekennzeichnet,**
daß er als Promotor die DNA-Sequenz der α-Glucosidase pl-Promotor-Region in verkürzter Form enthält, wobei mindestens die gesamte Region upstream des Nukleotids -867 und höchstens die gesamte Region upstream des Nukleotids -386 deletiert ist.

**2.** Expressionsvektor nach Anspruch 1,
**dadurch gekennzeichnet,**
daß in der verkürzten DNA-Sequenz der α-Glucosidase pl-Promotor-Region die gesamte Sequenz upstream des Nukleotids -427 deletiert ist.

**3.** Expressionvektor nach einem der Ansprüche 1 oder 2,
**dadurch gekennzeichnet,**
daß er zusätzlich ein positives Regulatorgen (MALp-Gen) enthält.

**4.** Expressionsvektor nach Anspruch 3,
**dadurch gekennzeichnet,**
daß er als positives Regulatorgen das MAL2-$8^c$p-Gen enthält.

**5.** Expressionsvektor nach einem der Ansprüche 1 bis 4,
**dadurch gekennzeichnet,**
daß er bereits als ein zu exprimierendes Fremdgen das Gen für die α-Glucosidase PI enthält.

**6.** Expressionsvektor YEp/S3, enthaltend eine α-Glucosidase-Expressionskassette, wie in Figur 3D dargestellt.

**7.** Expressionsvektor YEp/S4, enthaltend eine α-Glucosidase-Expressionskassette, wie in Figur 3E dargestellt.

**8.** Expressionsvektor YEp/5C6b3, enthaltend eine α-Glucosidase-Expressionskassette, wie in Figur 3F dargestellt.

**9.** Verfahren zur regulierbaren Herstellung von homologen und heterologen Proteinen oder proteinhaltigen Genprodukten in eukaryontischen Wirtszellen,
**dadurch gekennzeichnet,**
daß man in einen eukaryontischen Expressionsvektor gemäß den Ansprüchen 1 bis 4 eine für das gewünschte Protein oder Genprodukt kodierende DNA-Sequenz insertiert, den erhaltenen Vektor oder einen Expressionsvektor nach einem der Ansprüche 5 bis 8 durch Transformation in eine geeignete Wirtszelle einbringt, die Zellen kultiviert und das gebildete Protein oder Genprodukt aus den Zellen oder ihrem Nährmedium isoliert.

**10.** Verfahren nach Anspruch 9,
**dadurch gekennzeichnet,**
daß man einen Expressionsvektor nach den Ansprüchen 1 oder 2 und 6 oder 7 verwendet und in die Wirtszelle zusätzlich ein Plasmid oder integrierendes DNA-Fragment einbringt, das ein positives Regulatorgen enthält.

**11.** Verfahren nach Anspruch 10,
**dadurch gekennzeichnet,**
daß man zusätzlich ein das MAL2-$8^c$p-Gen enthaltendes plasmid oder integrierendes DNA-Fragment einbringt.

**12.** Verfahren nach einem der Ansprüche 9 bis 11,
**dadurch gekennzeichnet,**
daß man die DNA-Sequenz für α-Glucosidase PI als dem gewünschten Genprodukt insertiert.

## Claims

1. Eukarytoic expression vector, containing a regulstable promotor inserted into a eukaryotic DNA, characterised in that, as promotor, it contains the DNA sequence of the α-glucosidase pI promotor region in shortened form, whereby at least the whole region upstream of the nucleotide -867 and at most the whole region upstream of the nucleotide -386 is deleted.

2. Expression vector according to claim 1, characterised in that, in the shortened DNA sequence of the α-glucosidase pI promotor region, the whole sequence upstream of the nucleotide -427 is deleted.

3. Expression vector according to one of claims 1 or 2, characterised in that it additionally contains a positive regulator gene (MALp gene).

4. Expression vector according to claim 3, characterised in that it contains the MAL2-8$^c$p gene as positive regulator gene..

5. Expression vector according to one of claims 1 to 4, characterised in that, as a foreign gene to be expressed, it already contains the gene for the α-glucosidase pI.

6. Expression vector YEp/S3, containing an α-glucosidase expression cassette as illustrated in Fig. 3D.

7. Expression vector YEp/S4, containing an α-glucosidase expression cassette as illustrated in Fig. 3E.

8. Expression vector YEp/5C6b3, containing an α-glucosidase expression cassette as illustrated in Fig. 3F.

9. Process for the regulatable production of homologous and heterologous proteins or protein-containing gene products in eukaryotic cells, characterised in that one inserts into a eukaryotic expression vector according to claims 1 to 4 a DNA sequence coding for the desired protein or gene product, introduces the vector obtained or an expression vector according to one of claims 5 to 8 by transformation into a suitable host cell, cultures the cells and isolates the protein or gene product formed from the cells or their culture medium.

10. Process according to claim 9, characterised in that one uses an expression vector according to claims 1 or 2 and 6 or 7 and additionally introduces into the host cell a plasmid or integrating DNA fragment which contains a positive regulator gene.

11. Process according to claim 10, characterised in that one additionally introduces a plasmid containing the MAL2-8$^c$p gene or integrating DNA fragment.

12. Process according to one of claims 9 to 11, characterised in that one inserts the DNA sequence for α-glucosidase pI as the desired gene product.

## Revendications

1. Vecteur d'expression eucaryotique, contenant un promoteur régulable inséré dans un ADN eucaryotique, caractérisé en ce qu'il contient code promoteur la séquence d'ADN de la région promotrice de l'α-glucosidase pI sous forme raccourcie, au moins toute la région en amont du nucléotide -867 et au plus toute la région en amont du nucléotide -386 étant délétées.

2. Vecteur d'expression selon la revendication 1, caractérisé en ce que toute la séquence en amont du nucléotide -427 est délétée dans la séquence d'ADN raccourcie de la région promotrice de l'α-glucosidase pI.

3. Vecteur d'expression selon l'une des revendications 1 ou 2, caractérisé en ce qu'il contient en outre un gène régulateur positif (gène MALp).

4. Vecteur d'expression selon la revendication 3, caractérisé en ce qu'il contient comme gène régulateur positif le gène MAL2-8$^c$p.

5. Vecteur d'expression selon l'une des revendications 1 à 4, caractérisé en ce qu'il contient déjà comme gène étranger à exprimer le gène de l'α-glucosidase pI.

6. Vecteur d'expression YEp/S3, contenant une cassette d'expression d'α-glucosidase comme représenté sur la figure 3D.

7. Vecteur d'expression YEp/S4, contenant une cassette d'expression d'α-glucosidase comme représenté sur la figure 3E.

8. Vecteur d'expression YEp/5C6b3, contenant une cassette d'expression d'α-glucosidase comme représenté sur la figure 3F.

9. Procédé de préparation régulable de protéines homologues et hétérologues ou de produits de gènes contenant des protéines homologues et hétérologues dans des cellules hôtes eucaryotiques, caractérisé en ce que l'on insère dans un vecteur d'expression eucaryotique selon les revendications 1 à 4 une séquence d'ADN codant la protéine souhaitée ou le produit de gène souhaité, on introduit le vecteur obtenu ou un vecteur d'expression selon l'une des revendications 5 à 8 par transformation dans une cellule hôte appropriée, on cultive les cellules et on isole la protéine formée ou le produit de gène formé à partir des cellules ou de leur milieu de culture.

10. Procédé selon la revendication 9, caractérisé en ce que l'on utilise un vecteur d'expression selon les revendications 1 ou 2 et 6 ou 7 et on introduit en plus dans la cellule hôte un plasmide ou fragment d'ADN intégrant qui contient un gène régulateur positif.

11. Procédé selon la revendication 10, caractérisé en ce que l'on introduit en plus un plasmide ou fragment d'ADN intégrant contenant le gène <u>MAL2-8$^c$p</u>.

12. Procédé selon l'une des revendications 9 à 11, caractérisé en ce que, comme produit de gène souhaité, on insère la séquence d'ADN de l'α-glucosidase pI.

Fig. 1a

DNA-Sequenz des α-Glucosidase pI-Gens (GLUCPI) im Vergleich zum MAL6S Maltasegen, Konsensussequenz

```
         1                                                                  <------------Maltosepermease|     100
GLUCPI   .......... .......... .......GAT CTCATCTAAG TGTGAGTCGT TCCTGTCTTT TTTTCTGTTT ATTAATGAGG ATAATCCCTT CATAGTTAA.
MAL6S    GAATTCGGTA GCGTTCACGC CATTCTCGAT CTCATCTAAG TGTGAGTCGT TCCTGTCTTT TTTTCTGTTT ATTAATGAGG ATAATCCCTT CATAGTTAAC
CONS.    ---------- ---------- -------GAT CTCATCTAAG TGTGAGTCGT TCCTGTCTTT TTTTCTGTTT ATTAATGAGG ATAATCCCTT CATAGTTAA-

         101                                        <---------------|                                         ScaI
GLUCPI   TTAATAGTCT TGGATGTAAT TCTTATTGTT ATACTGAATA CGCTAAAACC ACTCACAACA AGTATGGAGT ATATTGTGTC TCTTTATATA CTGAGTACTT
MAL6S    TTAATAGTCT TGGATGTAAT TCTTATTGTT ATACTGAATA CGCTAAAACC ACTCACAACA AGTATGGAGT ATATTGTGTC TCTTTATATG CTGAGTACTT
CONS.    TTAATAGTCT TGGATGTAAT TCTTATTGTT ATACTGAATA CGCTAAAACC ACTCACAACA AGTATGGAGT ATATTGTGTC TCTTTATAT- CTGAGTACTT

         201                                                                                                  300
GLUCPI   ATG.CAATAT GCGCTCACTC AGGATGAAAT GTACACAGCC GAAAGTATAT TGAAAGCTGC CT.CTGTGGA AA.C.TTCTA T.C.TAATGT TGTCTCCAGA
MAL6S    ATGCCAATAT GCGCTCACTC AGGATGAAAT GTACACAGCC GAAAGTATAT T.AAAGCTGC CTGCTGTGCA AAGCGTTCTA TGCATAATGT TGTCTCCACA
CONS.    ATG-CAATAT GCGCTCACTC AGGATGAAAT GTACACAGCC GAAAGTATAT T-AAAGCTGC CT-CTGTG-A AA-C-TTCTA T-C-TAATGT TGTCTCCA-A

         301                                                                                                  400
GLUCPI   TGTAGACTAT GAGGCCTGAA GAAGTCTTTA AGTACCTGTT GGAGAGTAT. AAGGAGACTG CTACAACAAC GTCTTCCCCA CAAAAATTAT GTGGAGGCCG
MAL6S    TGTAGACTAT .AGGCCTGAA GAA.TCTTTA GGCACCTGTT GGAGAGTATG AAGGAGACTG CTACAACAAC GTCTTCCCCA CAAAAATTAT GTGGAGGCCG
CONS.    TGTAGACTAT -AGGCCTGAA GAA-TCTTTA -G-ACCTGTT GGAGAGTAT- AAGGAGACTG CTACAACAAC GTCTTCCCCA CAAAAATTAT GTGGAGGCCG
           -867               |
         500
GLUCPI   GTATGATACC TGCACAAACG TTAAGTTACA CATGAAAAAG AGACTGACAT AACTTTGATC TCTGAAAATA TGTTTTCCCC TG.AGTAGCT TCACTGCTTG
MAL6S    GTATGATACC TGCACAAACG TTAAGTTACA CATGAAAAAG AAACTGACAT AACTATGATC TCTGAAAATA TGTTTTCCCC TGGAGTAGCT TCACTGCTTG
CONS.    GTATGATACC TGCACAAACG TTAAGTTACA CATGAAAAAG A-ACTGACAT AACT-TGATC TCTGAAAATA TGTTTTCCCC TG-AGTAGCT TCACTGCTTG
             |---REPEAT-1----------->
         501                                                                                                  600
GLUCPI   GATACCAATA CGAATAGACC TTGGCTATAG TAAGTTGCAT CTGTACCGTA GAGATACCTG CACAAACGTT AAGTTACACA TGAAAAAGAG ACTGACGTAA
MAL6S    GATACCAATA CGAATAGACC TTGGCTATAG TAAGTTGCAT CTGTACCGTA GA........ .......... .......... .......... ..........
CONS.    GATACCAATA CGAATAGACC TTGGCTATAG TAAGTTGCAT CTGTACCGTA GA-------- ---------- ---------- ---------- ----------
                                                                 |---REPEAT-2----------->
```

Fortsetzung Fig. 1a (Seite 2)

```
        601                                                                                                         700
GLUCPI  TTTTGATCTC TGAAAATATG TTTTCCCCTG AGTAGCTTCA CTGCTTGGAT ACCAATACGA ATAGACCTTG GCTATAGTAA GTTGCATCTG TACCGTAGAG
MAL6S   .......... .......... .......... .......... .......... .......... .......... .......... .......... ..........
CONS.   ---------- ---------- ---------- ---------- ---------- ---------- ---------- ---------- ---------- ----------

        701                                                                                                         800
GLUCPI  ATACCTGCAC AAACGTTAAG TTACACATGA AAAAGAGACT GACGTAATTT TGATCTCTGA AAATATGTTT TCCCCTGAGT AGCTTCACTG CTTGGATACC
MAL6S   .......... .......... .......... .......... .......... .......... .......... .......... .......... ..........
CONS.   ---------- ---------- ---------- ---------- ---------- ---------- ---------- ---------- ---------- ----------
        --REPEAT-3------------>
        801                                          -427                                          SspI         900
GLUCPI  AATACGAATA GACCTTGGCT ATAGTAAGTT GCATCTGTAC CGTAGAGATT CTTGCAACCT CGCTT.AAAC TCTCGCTTTT ACA.TAATAT TTCTCCTTAT
MAL6S   .......... .......... .......... .......... ......GATT CTTGCAAC.T CGCTTGAAAC TCTCGCTTTT AGAGTAATAT TTCTCCTTAT
CONS.   ---------- ---------- ---------- ---------- ------GATT CTTGCAAC-T CGCTT-AAAC TCTCGCTTTT A-A-TAATAT TTCTCCTTAT

        901                                                                                                        1000
GLUCPI  TGCGCGCTTC GTTGAAAATT TCGCTAAACA CGGGGTTTAA GTTTAAGTTT ACAGGATTTA TCCGGAAGTT TTCG.CGGAC CCCACACAAT T.AAGAATTG
MAL6S   TGCGCGCTTC GTTGAAAATT TCGCTAAACA C.GGGTTTAA GTTAAAGTTT ACAGGATTTA TCCGGAAATT TTCGACGGAC CCCACACAAT TGAAGAATTG
CONS.   TGCGCGCTTC GTTGAAAATT TCGCTAAACA C-GGGTTTAA GTT-AAGTTT ACAGGATTTA TCCGGAA-TT TTCG-CGGAC CCCACACAAT T-AAGAATTG

        1001                                                                                                       1100
GLUCPI  GCTCGAAGAG TGATAACGCA TACTTTTCTT TTCTTTTTTT TAGTTCCTAG CGTACCTAAC GTAGGTAACA TGATTTGGAT CGT.GGGATG ATACAAACAA
MAL6S   GCTCGAAGAG TGATAACGCA TACTTTTCTT TTC.TTTTTT TAGTTCCTAG CGTACCTAAC GTAGGTAACA TGATTTGGAT CGTCGGGATG ATACAAACAA
CONS.   GCTCGAAGAG TGATAACGCA TACTTTTCTT TTC-TTTTTT TAGTTCCTAG CGTACCTAAC GTAGGTAACA TGATTTGGAT CGT-GGGATG ATACAAACAA

        1101                                                                                                       1200
GLUCPI  CGTAAGATGA GTAGTTCCTT CCTCAATTCT TCTTTCAGCA TCATTTTCTT GAGGCGCTCT GGGCAAGGTA TAAAAAGTTC CATTAATACG TCTCTAAAAA
MAL6S   CGTAAGATGA GTAGTTCCTT CCTCAATTCT TCTTGCAGCA TCATTTTCTT GAGGCGCTCT GGGCAAGGTA TAAAAAGTTC CATTAATACG TCTCTAAAAA
CONS.   CGTAAGATGA GTAGTTCCTT CCTCAATTCT TCTT-CAGCA TCATTTTCTT GAGGCGCTCT GGGCAAGGTA TAAAAAGTTC CATTAATACG TCTCTAAAAA
```

Fortsetzung Fig. 1a (Seite 3)

```
                                                           ┌┐──────────>                     |----------MALTASE------>
        1201                                               ||                              +1
GLUCPI  ATTAAACCAT CTATCTCTTA AGCAGTTTTT TTGATAATCT CAAATGTACA TCAGTCAAGC GTAACTAAAT TACATAAATG ACTATTTCTG ATCATCCAGA
MAL6S   ATTAAATCAT CCATCTCTTA AGCAGTTTTT TTGATAATCT CAAATGTACA TCAGTCAAGC GTAACTAAAT TACATAAATG ACTATTTCTG ATCATCCAGA
CONS.   ATTAAA-CAT C-ATCTCTTA AGCAGTTTTT TTGATAATCT CAAATGTACA TCAGTCAAGC GTAACTAAAT TACATAAATG ACTATTTCTG ATCATCCAGA

        1301                                                                                                  1400
GLUCPI  AACAGAACCA AAGTGGTGGA AAGAGGCCAC AATCTATCAA ATTTACCCAG CAAGTTTTAA AGACTCCAAT AACGATGGCT GGGGTGATTT AAAAGGTATC
MAL6S   AACAGAACCA AAGTGGTGGA AAGAGGCCAC AATCTATCAA ATTTACCCAG CAAGTTTTAA AGACTCCAAT AACGATGGCT GGGGTGATTT AAAAGGTATC
CONS.   AACAGAACCA AAGTGGTGGA AAGAGGCCAC AATCTATCAA ATTTACCCAG CAAGTTTTAA AGACTCCAAT AACGATGGCT GGGGTGATTT AAAAGGTATC

        1401                                                                                                  1500
GLUCPI  ACTTCCAAGT TGCAGTATAT TAAAGATCTT GGCGTTGATG CTATTTGGGT TTGTCCGTTT TATGACTCTC CTCAACAAGA TATGGGGTAT GATATATCTA
MAL6S   ACTTCCAAGT TGCAGTATAT TAAAGATCTT GGCGTTGATG CTATTTGGGT TTGTCCGTTT TATGACTCTC CTCAACAAGA TATGGGGTAT GATATATCCA
CONS.   ACTTCCAAGT TGCAGTATAT TAAAGATCTT GGCGTTGATG CTATTTGGGT TTGTCCGTTT TATGACTCTC CTCAACAAGA TATGGGGTAT GATATATC-A

        1501                                                                                                  1600
GLUCPI  ACTACGAAAA GGTCTGGCCC ACATACGGTA CCAACGAGGA CTGTTTTGAG CTAATTGACA AGACTCATAA GCTGGGTATG AAATTCATCA CCGATTTGGT
MAL6S   ACTACGAAAA GGTCTGGCCC ACATACGGTA CCAATGAGGA CTGTTTTGAG CTAATTGACA AGACTCATAA GCTGGGTATG AAATTCATCA CCGATTTGGT
CONS.   ACTACGAAAA GGTCTGGCCC ACATACGGTA CCAA-GAGGA CTGTTTTGAG CTAATTGACA AGACTCATAA GCTGGGTATG AAATTCATCA CCGATTTGGT

        1601                                                                                                  1700
GLUCPI  TATCAACCAC TGTTCTACAG AACACGAATG GTTCAAAGAG AGCAGATCCT CGAAGACCAA TCCGAAGCGT GACTGGTTCT TCTGGAGACC TCCTAAGGGT
MAL6S   TATCAACCAC TGTTCTACAG AACACGAATG GTTCAAAGAG AGCAGATCCT CGAAGACCAA TCCGAAGCGT GACTGGTTCT TCTGGAGACC TCCTAAGGGT
CONS.   TATCAACCAC TGTTCTACAG AACACGAATG GTTCAAAGAG AGCAGATCCT CGAAGACCAA TCCGAAGCGT GACTGGTTCT TCTGGAGACC TCCTAAGGGT

        1701                                                                                                  1800
GLUCPI  TATGACGCCG AAGGCAAGCC AATTCCTCCA AACAATTGGA AATCTTTCTT TGGTGGTTCA GCTTGGACTT TTGATGAAAC TACAAATGAA TTTTACCTCC
MAL6S   TATGACGCCG AAGGCAAGCC AATTCCTCCA AACAATTGGA AATCTTTCTT TGGTGGTTCA GCTTGGACTT TTGATGAAAC TACAAATGAA TTTTACCTCC
CONS.   TATGACGCCG AAGGCAAGCC AATTCCTCCA AACAATTGGA AATCTTTCTT TGGTGGTTCA GCTTGGACTT TTGATGAAAC TACAAATGAA TTTTACCTCC
```

Fortsetzung Fig. 1a (Seite 4)

```
         1801                                                                                                  1900
GLUCPI   GTTTGTTTGC GAGTCGTCAA GTTGACTTGA ATTGGGAGAA TGAAGACTGC AGAAGGGCAA TCTTTGAAAG TCCTGTTGGA TTTTGGCTGG ACCATGGTGT
MAL6S    GTTTGTTTGC GAGTCGTCAA GTTGACTTGA ATTGGGAGAA TGAAGACTGC AGAAGGGCAA TCTTTGAAAG TGCTGTTGGA TTTTGGCTGG ACCATGGTGT
CONS.    GTTTGTTTGC GAGTCGTCAA GTTGACTTGA ATTGGGAGAA TGAAGACTGC AGAAGGGCAA TCTTTGAAAG T-CTGTTGGA TTTTGGCTGG ACCATGGTGT

         1901                                                                                                  2000
GLUCPI   AGATGGTTTT AGAATCGATA CCGCTGGTTT GTATTCGAAA CGTCCTGGTT TACCAGATTC CCCAATTTTT GACAAAACCT CGAAATTACA ACATCCAAAT
MAL6S    AGATGGTTTT AGAATCGATA CCGCTGGTTT GTATTCGAAA CGTCCTGGTT TACCAGATTC CCCAATTTTT GACAAAACCT CGAAATTACA ACATCCAAAT
CONS.    AGATGGTTTT AGAATCGATA CCGCTGGTTT GTATTCGAAA CGTCCTGGTT TACCAGATTC CCCAATTTTT GACAAAACCT CGAAATTACA ACATCCAAAT

         2001                                                                                                  2100
GLUCPI   TGGGGGTCTC ACAATGGTCC TAGGATTCAT GAATATCATC AAGAACTACA CAGATTTATG AAAAACAGGG TGAAAGATGG TAGAGAAATA ATGACAGTCG
MAL6S    TGGGGGTCTC ACAATGGTCC TAGGATTCAT GAATATCATC AAGAACTACA CAGATTTATG AAAAACAGGG TGAAAGATGG TAGAGAAATA ATGAGAGTCG
CONS.    TGGGGGTCTC ACAATGGTCC TAGGATTCAT GAATATCATC AAGAACTACA CAGATTTATG AAAAACAGGG TGAAAGATGG TAGAGAAATA ATGA-AGTCG

         2101                                                                                                  2200
GLUCPI   GTGAAGTTGC CCATGGAAGT GATAATGCTT TATACACCAG TGCAGCTAGA TACGAAGTCA GCGAAGTTTT CTCCTTCACG CACGTTGAAG TTGGTACCTC
MAL6S    GTGAAGTTGC CCATGGAAGT GATAATGCTT TATACACCAG TGCAGCTAGA TACGAAGTCA GCGAAGTTTT CTCCTTCACG CACGTTGAAG TTGGTACCTC
CONS.    GTGAAGTTGC CCATGGAAGT GATAATGCTT TATACACCAG TGCAGCTAGA TACGAAGTCA GCGAAGTTTT CTCCTTCACG CACGTTGAAG TTGGTACCTC

         2201                                                                                                  2300
GLUCPI   GCCATTTTTC CGTTATAACA TAGTGCCCTT CACCTTGAAA CAATGGAAAG AAGCCATTGC ATCGAACTTT TTGTTCATTA ACGGTACTGA TAGTTGGGCT
MAL6S    GCCATTTTTC CGTTATAACA TAGTGCCCTT CACCTTGAAA CAATGGAAAG AAGCCATTGC ATCGAACTTT TTGTTCATTA ACGGTACTGA TAGTTGGGCT
CONS.    GCCATTTTTC CGTTATAACA TAGTGCCCTT CACCTTGAAA CAATGGAAAG AAGCCATTGC ATCGAACTTT TTGTTCATTA ACGGTACTGA TAGTTGGGCT

         2301                                                                                                  2400
GLUCPI   ACCACCTACA TCGAGAATCA CGATCAAGCC CGGTCAATTA CGAGATTTGC TGACGATTCG CCAAAGTACC GTAAAATATC TGGTAAGCTG TTAACATTGC
MAL6S    ACCACCTACA TCGAGAATCA CGATCAAGCC CGGTCAATTA CGAGATTTGC TGACGATTCG CCAAAGTACC GTAAAATATC TGGTAAGCTG TTAACATTGC
CONS.    ACCACCTACA TCGAGAATCA CGATCAAGCC CGGTCAATTA CGAGATTTGC TGACGATTCG CCAAAGTACC GTAAAATATC TGGTAAGCTG TTAACATTGC
```

Fortsetzung Fig. 1a (Seite 5)

```
        2401                                                                                                      2500
GLUCPI  TAGAATGTTC ATTGACAGGT ACGTTGTATG TCTATCAAGG TCAGGAGATA GGCCAGATCA ATTTCAAGGA ATGGCCTATT GAAAAGTATG AGGACGTTGA
MAL6S   TAGAATGTTC ATTGACAGGT ACGTTGTATG TCTATCAAGG TCAGGAGATA GGCCAGATCA ATTTCAAGGA ATGGCCTATT GAAAAGTATG AGGACGTTGA
CONS.   TAGAATGTTC ATTGACAGGT ACGTTGTATG TCTATCAAGG TCAGGAGATA GGCCAGATCA ATTTCAAGGA ATGGCCTATT GAAAAGTATG AGGACGTTGA

        2501                                                                                                      2600
GLUCPI  TGTGAAAAAC AACTACGAGA TTATCAAAAA AAGTTTTGGT AAAAACTCGA AGGAAATGAA GGATTTTTTT AAAGGAATCG CCCTACTTTC TAGAGATCAT
MAL6S   TGTGAAAAAC AACTACGAGA TTATCAAAAA AAGTTTTGGT AAAAACTCGA AGGAAATGAA GGATTTTTTT AAAGGAATCG CCCTACTTTC TAGAGATCAT
CONS.   TGTGAAAAAC AACTACGAGA TTATCAAAAA AAGTTTTGGT AAAAACTCGA AGGAAATGAA GGATTTTTTT AAAGGAATCG CCCTACTTTC TAGAGATCAT

        2601                                                                                                      2700
GLUCPI  TCGAGAACTC CCATGCCATG GACGAAAGAT AAGCCCAATG CTGGATTTAC TGGCCCAGAT GTTAAACCTT GGTTTTTCTT GAATGAATCT TTTGAGCAAG
MAL6S   TCGAGAACTC CCATGCCATG GACGAAAGAT AAGCCCAATG CTGGATTTAC TGGCCCAGAT GTTAAACCTT GGTTTTTCTT GAATGAATCT TTCGAGCAAG
CONS.   TCGAGAACTC CCATGCCATG GACGAAAGAT AAGCCCAATG CTGGATTTAC TGGCCCAGAT GTTAAACCTT GGTTTTTCTT GAATGAATCT TT-GAGCAAG

        2701                                                                                                      2800
GLUCPI  GAATCAATGT TGAGCAGGAA TCCAGAGATG ATGACTCAGT TCTCAATTTT TGGAAAAGGG CCTTGCAAGC CAGAAAGAAA TATAAGGAAC TTATGATTTA
MAL6S   GAATCAATGT TGAGCAGGAA TCCAGAGATG ATGACTCAGT TCTCAATTTT TGGAAAAGGG CCTTGCAAGC CAGAAAGAAA TATAAGGAAC TTATGATTTA
CONS.   GAATCAATGT TGAGCAGGAA TCCAGAGATG ATGACTCAGT TCTCAATTTT TGGAAAAGGG CCTTGCAAGC CAGAAAGAAA TATAAGGAAC TTATGATTTA

        2801                                                                                                      2900
GLUCPI  TGGTTACGAT TTCCAATTCA TTGATTTAGA CAGTGACCAG ATCTTTAGCT TCACTAAAGA GTACGGAGAC AAGACGCTGT TTGCTGCTTT GAATTTCAGT
MAL6S   TGGTTACGAT TTCCAATTCA TTGATTTAGA CAGTGACCAG ATCTTTAGCT TCACTAAAGA GTACGAAGAC AAGACGCTGT TTGCTGCTTT AAATTTCAGT
CONS.   TGGTTACGAT TTCCAATTCA TTGATTTAGA CAGTGACCAG ATCTTTAGCT TCACTAAAGA GTACG-AGAC AAGACGCTGT TTGCTGCTTT -AATTTCAGT

        2901                                                                                                      3000
GLUCPI  GGCGAAGAAA TTGAATTCAG CCTCCCAAGA GAAGGTGCTT CTTTATCTTT TATTCTTGGA AATTATGATG ATACTGACGT TTCCTCCAGA GTTTTGAAAC
MAL6S   GGCGAAGAAA TTGAATTCAG CCTCCCAAGA GAAGGTGCTT CTTTATCTTT TATTCTTGGA AATTATGATG ATACTGACGT TTCCTCCAGA GTTTTGAAAC
CONS.   GGCGAAGAAA TTGAATTCAG CCTCCCAAGA GAAGGTGCTT CTTTATCTTT TATTCTTGGA AATTATGATG ATACTGACGT TTCCTCCAGA GTTTTGAAAC
```

Fortsetzung Fig. 1a (Seite 6)

```
        3001                                                                                                       3100
GLUCPI  CATGGGAAGG TAGAATCTAC CTCGTCAAAT AAAATTAGTG CGGACTTTTT TTTAGCGCGT ACTTTAACGA AATAACAGAT GATTTTTCAC ATGATTTTTG
MAL6S   CATGGGAAGG TAGAATCTAC CTCGTCAAAT AAAATTAGTG CCGGCTTTTT TTTAGCGCGT ACTTTAACGA AATAACACAT GATTTTTCAC ATGATTTTTG
CONS.   CATGGGAAGG TAGAATCTAC CTCGTCAAAT AAAATTAGTG C-G-CTTTTT TTTAGCGCGT ACTTTAACGA AATAACA-AT GATTTTTCAC ATGATTTTTG

        3101                                                                                                       3200
GLUCPI  TTAGATAAAT TTTTTATATG TAAATGATGA TAGCGTAAAA GCACTGTTGA TAATTTGTTT CACCATTATG GGTAAATGTG TTTTTCTACA TGATCCACGT
MAL6S   TTAGATAAAT TTTTTATATG TAAATGATGA TAGCGTAAAA GCACTGTTGA TAATTTGTTT CACCATTATG GGTAAATGTG TTTTTCTACA TGACCCTCGT
CONS.   TTAGATAAAT TTTTTATATG TAAATGATGA TAGCGTAAAA GCACTGTTGA TAATTTGTTT CACCATTATG GGTAAATGTG TTTTTCTACA TGA-CC-CGT

        3201                                                                                                       3300
GLUCPI  TCATTATGAT ATTTAGCGTG TATATAAATG TGAATTCCAA ATTATTAATG AAGCATAAGA AGCACTATCC TTTCTCTTCG GATGAAAACA AGGGAGAAGA
MAL6S   TCATTATGCT ATTTAGCGTG TATATAAATG TGAATTC
CONS.   TCATTATG-T ATTTAGCGTG TATATAAATG TGAATTC

        3301                                                                                                       3400
GLUCPI  AACCTGTGCT GGTATTAATG CTGGAATGTC TTGCTAAGAA TCATACAAGG TGGTAGTTTT ATTTAATAAA GAAAAGAAAA GGACTAGATA TAAAAAGTGA

        3401
                                             SacI
GLUCPI  AATGAATATA AGATAGCGTT AAGAGATGCC CACAGTACT
```

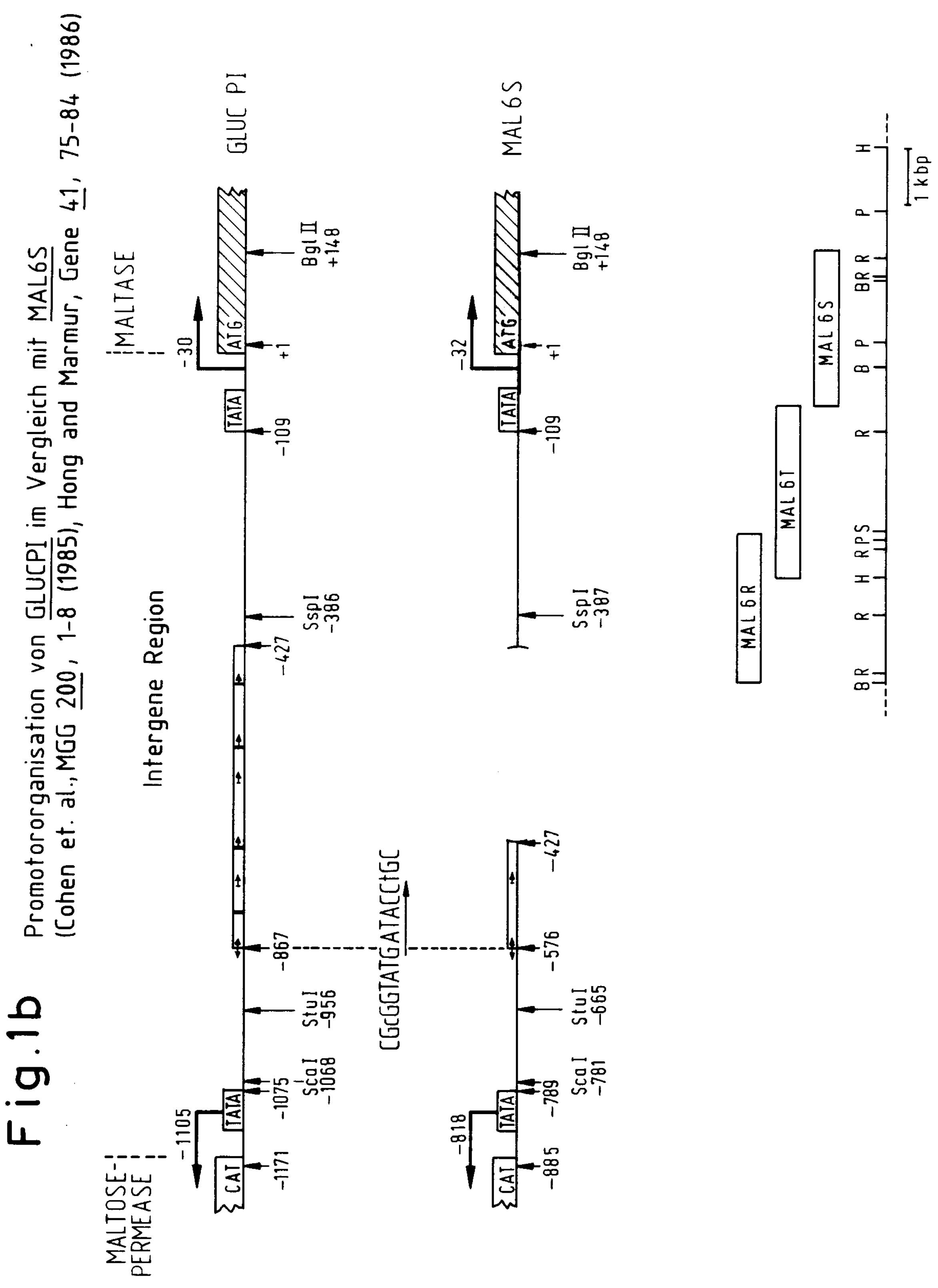


Fig.1b

Promotororganisation von GLUCPI im Vergleich mit MAL6S (Cohen et. al., MGG 200, 1-8 (1985), Hong and Marmur, Gene 41, 75-84 (1986)

Fig. 2

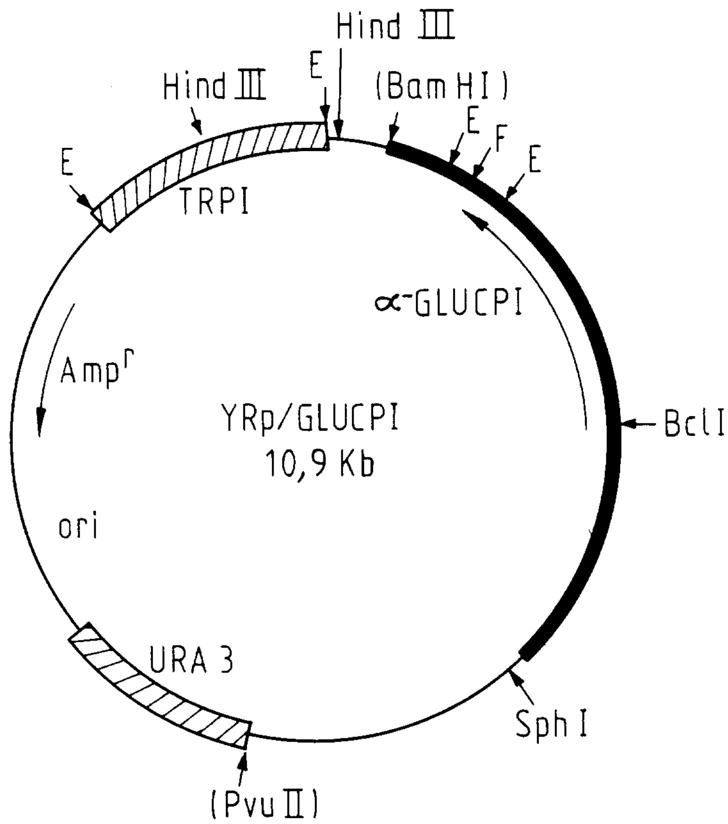
Hind III
E
Hind III
(Bam HI)
E
F
E
E
TRPI
α-GLUCPI
Amp^r
YRp/GLUCPI
10,9 Kb
BclI
ori
URA 3
Sph I
(Pvu II)

Fig.3

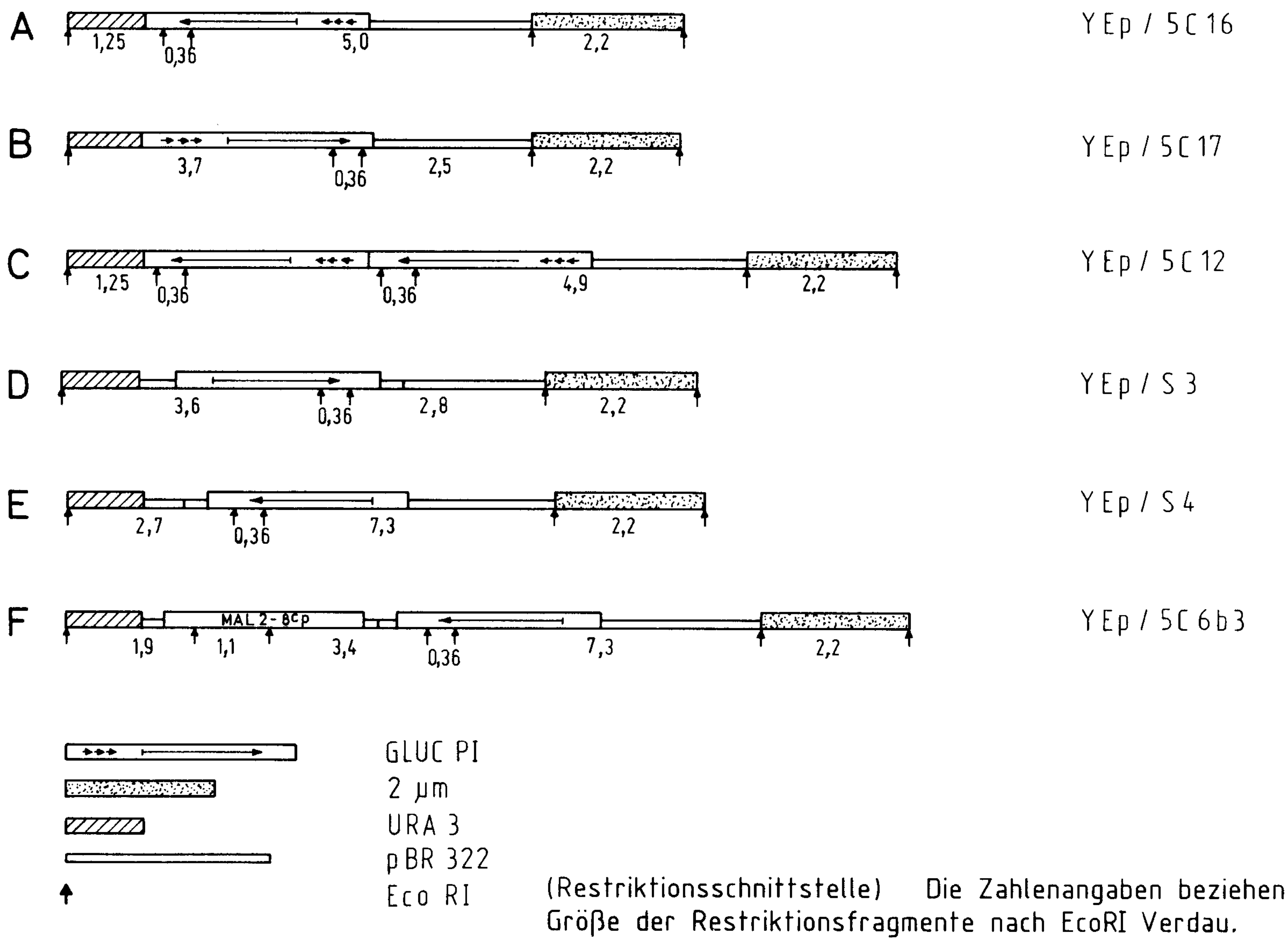


Die Zahlenangaben beziehen sich auf Größe der Restriktionsfragmente nach EcoRI Verdau.

Fig. 4

Derepression von α-Glucosidase pI in ABYSMAL 81 Transformanten unter Kontrolle des authentischen (A), verkürzten (B) und verkürzten MAL2-8$^c$p stimulierten Promotors (C) in Glucose Minimal Medium. (Y) Glucose Verbrauch

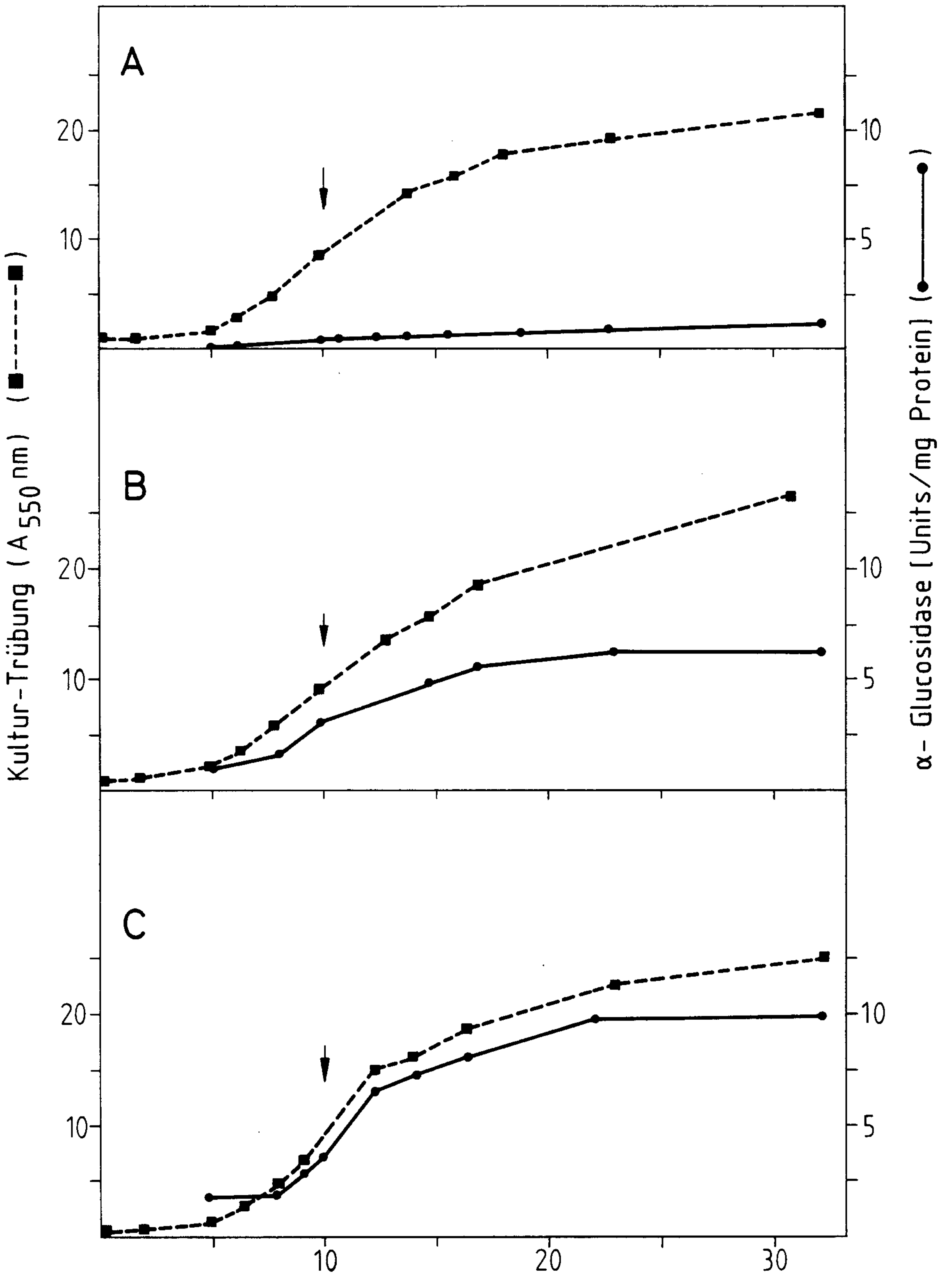

Fig.5

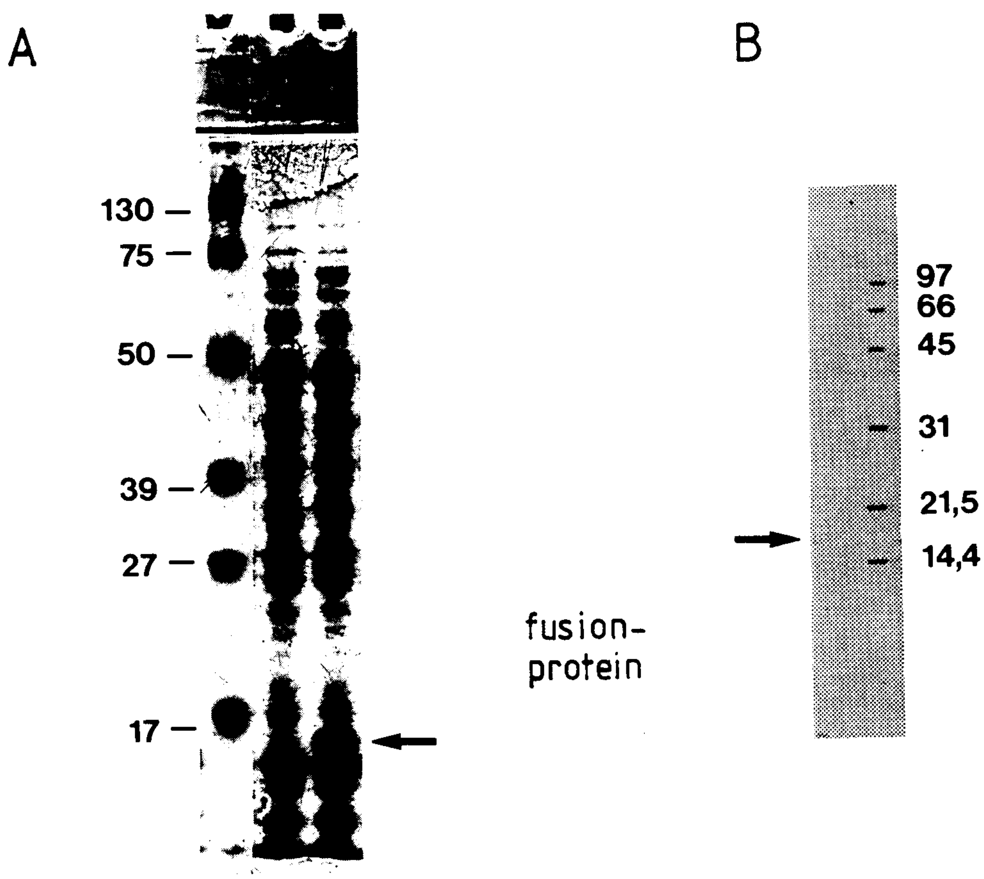


SDS-PAGE Analyse des α-Gluc. PI -env. Fusionsproteins in dem Wirtsstamm ABYSMAL 81 (A) Coomassie-Färbung, (B) Immunoblot Analyse

Fig. 6

```
1    GATCTTGAAT ATTTTCCTCT CCGAGATTTT CTCCGACAAT TGTTCCTCCG CGCAGCCAAC
61   GTTATTTCTC AGGTCCATCG ATTTTTTGTT TTTACCATAT TCCTTAAAGA CATACACAAG
121  GTTAGTAGGA GAGGAAACCC ATGGGGAGTA GTGCACGAAA AAGCTTAGAA AACCGTCCCT
181  GGCGTTTATT GCATAGAGAC ACATCTCTTT CCGTACAACG GAGCAAATAT CACAGAAGCG
241  GTACTCATCT ATGTTAGTAT CCTTGCGATT CCTCTCACCT ATCACAGAAT GTTATTCTAA
301  CACTACTAGA GATAGAAATT TAAGCAGAAC CGCCTTATTT TCAGAACGAA TTTCGCCAGC
361  TTAGTTTGCT CGAGGAAGGG GGGTGGGCGT GAATTTTTTT TTCTTGTGTC ATCCATTACT
421  CTCCCTCCTG AGTATGGGAG GAGAAGATTA TTCGAGTAGA ACGGAGATTT TTTTTTTTGG
481  CTTAATACGT GCCTATTCAC TGTCGATATT TCAATGAGAA GAACTGTACT GTTCGGGGGA
541  ATATTTAGAA GGAATTGGAT TCGATGGACA TTAGTGGCTT CTTTGTTACC TCATATTTTT
601  CGTTCTCATT GCAGCGCCCT TCCTCACCCG CGGGCGCTTT TATCCCGCCC CGGCTTTTAT
661  TGTCCGTTCC ATTGTATTCA ATAACTTAAA TACAACAGAA AATGATTACA GGATTCTTCT
721  CTCATAGAAA TATAAGCAAA CTTCAAATGG AACTGAGAAA AAAAATTTTC CTTCTATTGG
781  AATTTTACTT TACAAATGAC TTTGGCAAAA CAGGCATGCG ATTGTTGTCG CGTTCGTCGA
841  GTAAAGTGTG ACAGGAATAA ACCATGTAAT CGCTGCATTC AGCGCAATTT GAACTGCACT
901  TATCTTCAAC CGTTGAAAAA GAGAGGTCCA AAATCCATTA GAGCAGGAAG CTTAAAAAAA
961  ATAGCGGAAG TGCAGATGGT GAGTATGAAT AATAATATTA TGGCCGCTCC GGTGGTATGT
1021 AAGAAGGTTC CGAAAAACCT GATTGATCAA TGTTTGAGGT TGTATCATGA TAACTTATAT
1081 GTAATTTGGC CAATGCTTTC CTACGATGAT CTTCACAAGC TTCTAGAAGA GAAATACGAT
1141 GACCGTTGCG CCTACTGGTT TCTGGTATCC CTTTCGGCAG CTACTCTTAG CGACTTGCAA
1201 ATTGAAATAG AGTATGAGGA AGGAGTCACT TTTACTGGAG AGCAGTTGTG CACTCTTTGC
1261 ATGTTATCTC GGCAATTCTT TGACGACCTT AGTAACAGCG ACATATTTCG AATCATGACA
1321 TACTATTGTT TGCACCGTTG TTACGCGCAG TTTGCGGATA CGAGAACTTC ATATAGACTT
1381 TCTTGTGAAG CCGTGGGCCT CATCAAGATA GCTGGATTCC ATCGGGAAGA AACCTATGAA
1441 TTCCTTCCCT TCGGTGAACA ACAACTCAGA AGGAAAGTTT ACTATTTACT TCTTATGACA
```

1501 GAGAGATTTT ACGCTGTATA TATTAAGTGT GTCACGAGCC TAGATGCAAC AATAGCGCCA

1561 CCACTACCAG AGGTTGTAAC AGACCCTCGT CTTTCTCTAG AAAGCTTCCT TGAGGTGATT

1621 AGAGTTTTCA CTATACCAGG AAAGTGTTTT TATGATGCTT TGGCTACTAA CTGTGTCGAT

1681 GATTCCTGCA CCGAAGACTC TCTAAAAAGG ATATGGAACG AACTTCATAC CACATCACTT

1741 GATATAGAGC CATGGTCTTA CGGATACATC GATTTTCTGT TTTCGAGGCA TTGGGTCAGG

1801 ACACTAGCGT GGAAACTAGT ACTTCATATG AAAGGTATGC GGATGAATTT TCTTTCGAAT

1861 ACTAATAACA CACATATACC AGTCGAAATT GCTAGAGACA TGTTGGGAGA CACGTTTTTA

1921 ACTCCGAAAA ACCTGTATGA TGTACATGGT CCTGGAATAC CGATGAAGGC ATTAGAAATA

1981 GCCAATGCAT TGGTAGATGT CGTAAATAAG TATGATCACA ATATGAAGTT GGAAGCTTGG

2041 AATGTTTTGT ATGATGTATC CAAGTTTGTT TTTTCTCTGA AACATTGCAA TAATAAAATG

2101 TTCGACAGAT TTTCAACGAA ATGTCAAGGT GCCCTAATTA CTCTGCCCAT TTCTAAACCT

2161 TTGCAATTAA ATGATAACTC CAAAGATGAA GACGACATAA TTCCTTAATT TATTGTTCAC

2221 GCCGTTCACT TATACGAGAT AGATATACTG ATAGAGTGTG AGCGATATTC TTAAGTCTTC

2281 CTTTTCGAGG GTGTAAGAAG CTATGTTCAG GCGAGATTAT TCTACTCCTG CCTTACTTGT

2341 TTGTAATATT TAGTTCTGAT GGTCATGATA ATTCTATATA CAGTTACATT AAGTATATAC

2401 TTAAGCGGGC AGCTTACTAA TATAAATTTT GTGGCATTTT TGTTGGGATA TGAGAATCAT

2461 GTATCGTTGA TTTGCAAAGC GAATTTACGT TACCAGGAAT AGGGAATACT CTCTTGAATT

2521 CTAACATAAG CATATAAATG CTGAAAGAAT ACGTCAAAAA GTAAATTTAC AGAATTAAAA

2581 AAAATAATTG TTGCCGGAAC ATGAATAGAG TGTATCAACA TACAATATAA AAATTTAAGC

2641 ATTTTCATTA ACGGGAATGA AAAAATTGTA TATTAGTACT TCTTTTTGCA AGATATTTAA

2701 ACCTAGGACC CTCATCACAA TGATTCATTT AAATCATTAA CGTTTTTTCA GAACAAAATA

2761 ATAACCTCTA GTTGAAAAAA CTTCAAAGCC GCAACAAAAC CAAAAAAAAG TCATAATGTC

2821 GAGTAAAAAT AAACTCGATG TGTTAAGTAC TAGTTATATA AAATAAAATT AAATAACGCC

2881 GTATCTACCT ACTGGCTAAA AAAATCATTT GTTCACAACA GATGGGGTCT TCGCCCTTGA

2941 TCC